# EUROPEAN PATENT APPLICATION

(11) **EP 2 526 941 A1**
(43) Date of publication of application: **28.11.2012**
(21) Application number: 12169609.0
(22) Date of filing: 24.03.2006
(51) Int. Cl.: A61K 31/353, A61P 29/00

(54) **Anti-inflammatory Modalities**

(30) Priority: 24.03.2005 AU 2005901475; 06.04.2005 US 668609 P; 28.10.2005 AU 2005905985
(62) Divisional of application: 06721289.4
(71) Applicant: Marshall Edwards, Inc., San Diego, CA 92130 (US)
(72) Inventor: Walker, Catherine, Balmain, New South Wales 2041 (AU); Husband, Alan James, Pyrmont, New South Wales 2009 (AU); James, Michael, John, Dulwich, South Australia 5065 (AU)
(74) Representative: Wichmann, Hendrik

(57) **Abstract**

Anti-inflammatory modalities are described with reference to select isoflavonoid compounds, compositions containing same and the use of said compounds and/or compositions in treatment, particularly for the treatment of inflammatory diseases and related conditions.

## Description

This is a divisional application on the basis of the parent application European Patent Application 06 721 289.4 the content of which is incorporated herein by reference.

### Field of the Invention

The present invention relates to certain isoflavonoid compounds, compositions containing same and the use of said compounds and/or compositions in treatment, particularly for the treatment of inflammatory diseases and conditions. Inflammatory conditions include irritable bowel disease (IBD), for example: ulcerative colitis (UC), ulcerative proctitis, distal colitis; and/or Crohn's disease (CD), as well as other hepatointestinal syndromes including primary sclerosing cholangitis (PSC), primary biliary cirrhosis (PBC), autoimmune hepatitis (AIH) and irritable bowel syndrome (IBS).

### Background of the invention

UC causes inflammation of the inner lining of the large bowel (colon and rectum). CD causes inflammation of the full thickness of the bowel wall and may involve any part of the digestive tract from the mouth to the anus. CD can cause recurrent bowel obstruction, fistulae, abscess formation and sepsis as well as extra-intestinal manifestations such as arthritis. IBD often develops between the ages of 15-30, and about 13,000 Australians have UC and 10, 000 have CD. The Crohn's and Colitis Foundation of America estimates as many as 1,000,000 Americans have IBD costing directly and indirectly around $US552 billion annually. Furthermore, there is research to suggest that persons with IBD are more likely to develop colon cancer.

The cause of IBD is unknown. However, both syndromes would appear to be immunologically-mediated and the inflammatory process is influenced by environmental and genetic factors. Medical therapy aims to control the inflammatory process, and is administered for active and chronic disease, as well as remission maintenance. No management strategy is totally effective, but current therapy is targeted at reducing inflammation and/or the immune response using anti-inflammatories (corticosteroids, aminosalicylates), immunosuppressives, immunotherapies or surgery. The goals of treatment are to induce and then maintain remission of disease, minimising the side-effects which accompany the therapies. Up to 80% of patients with UC and 35% in those with CD relapse within a year following the induction of remission (Podolsky, D. K. (2002) "The current future understanding of inflammatory bowel disease." Best Practice & Research in Clinical Gastroenterology 16(6): 933-43). Additionally, none of the existing therapies are without significant side effects. The 'holy grail' in IBD drug development is therefore a non-toxic agent which will maintain remission of disease (Feagan 2003 "Maintenance therapy for inflammatory bowel disease" The American Journal of Gastroenterology 98 (12, Supplement 1): S6-S17).

Primary biliary cirrhosis (PBC), autoimmune hepatitis (AIH) and primary sclerosing cholangitis (PSC) are chronic liver diseases that are likely to have an autoimmune basis to their pathogenesis. PSC appears to be associated with UC. In PSC and PBC, the bile ducts become inflammed, scarred and eventually blocked, causing cholestasis, hepatocellular injury and in many cases liver failure.

Irritable Bowel Syndrome (IBS) is part of a spectrum of diseases known as Functional Gastrointestinal Disorders which include diseases such as non-cardiac chest pain, non-ulcer dyspepsia, and chronic constipation or diarrhoea. These diseases are all characterised by chronic or recurrent gastrointestinal symptoms for which no structural or biochemical cause can be found. IBS affects between 25 and 55 million people in the USA.

The prevalence of IBS in the general population of Western countries varies from 6 to 22%. IBS affects 14-24% of women and 5-19% of men. The prevalence is similar in Caucasians and African Americans, but appears to be lower in Hispanics. Although several studies have reported a lower prevalence of IBS among older people, the present studies do not allow to definitely conclude whether or not an age disparity exists in IBS. In non-Western countries such as Japan, China, India, and Africa IBS also appears to be very common.

Accordingly there is a need for new therapies in the treatment of inflammation and related diseases and conditions and new and improved agents and compounds useful for same.

### Summary of the Invention

Surprisingly the inventors have found that compounds of formula (I) 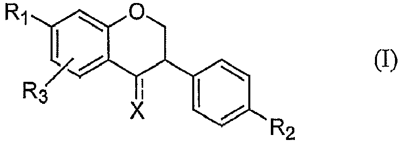 and salts thereof wherein:
- R₁ and R₂: are independently hydroxy, alkoxy or acyloxy,
- R₃: is hydroxy, alkoxy, alkyl or halo, and
- X: is either =O or hydrogen and hydroxy
are particularly useful in the treatment of inflammatory diseases and cardiovascular diseases.

Preferably R₁ and R₂ in compounds of formula (I) represent hydroxy.

Preferably R₃ in compounds of formula (I) represents methyl, bromo, chloro or hydroxy.

In a preferred embodiment X is =O and the compounds of the invention are isoflavan-4-ones of the formula (Ia): 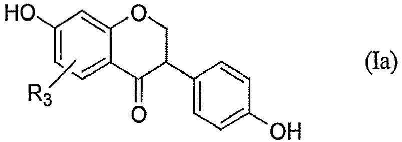 wherein
- R3: is 5-alkyl, 6-halo or 8-halo,
more preferably
- R3: is 5-methyl, 6-chloro or 8-bromo.

In another preferred embodiment X is hydrogen and hydroxy and the compounds of the invention are isoflavan-4-ols of the formula (Ib): 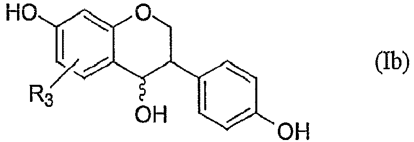 wherein
- R3: is 8-alkyl or 8-hydroxy,
more preferably
- R3: is 8-methyl, or 8-hydroxy.

In still another preferred embodiment the compounds are 8-substituted isoflavonoid compounds of the formula (Ic): 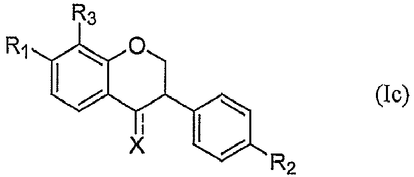 and salts thereof wherein:
- R₁ and R₂: are independently hydroxy, alkoxy or acyloxy,
- R₃: is hydroxy, alkyl or halo, and
- X: is either =O or hydrogen and hydroxy,
more preferably
- R₁ and R₂: are hydroxy, and
- R₃: is hydroxy, methyl or bromo.

Particularly preferred isoflavonoid compounds of formula (I) and pharmaceutically acceptable salts thereof are selected from compounds 1 to 5:

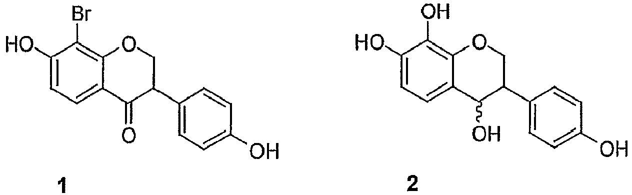

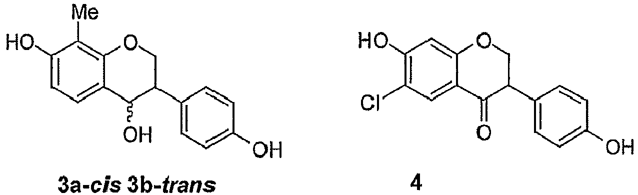

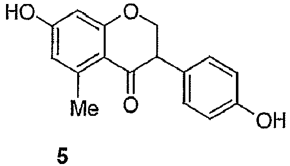

According to another aspect of the present invention there is provided the use of one or more compounds of formula (I) as an anti-inflammatory agent or cardiovascular agent.

The inflammatory disorders include pain, oedema and erythema associated with inflammation in general, inflammatory disorders including osteoarthritis, inflammatory bowel disease (ulcerative colitis and Crohn's disease), ulcerative proctitis, distal colitis, autoimmune disorders (SLE, rheumatoid arthritis, glomerulonephritis), asthma and diseases involving pulmonary inflammation, cardiovascular disorders including atherosclerosis, hypertension and lipid dyscrasias and disorders associated with estrogen receptor activation. That is the compounds are useful for the treatment of pain associated with inflammation. In a preferred embodiment, the treatment of the inflammatory disorders is absent cardiovascular side effects, cardioprotective and/or is gut protective.

According to another aspect of the present invention there is provided the use of a thromboxane synthase inhibitor for the manufacture of a medicament for the treatment of inflammation, including pain associated with inflammation. Preferably the treatment is cardioprotective and/or gut protective.

According to another aspect of the invention there is provided a pharmaceutical agent comprising a compound of formula (I) for the treatment of inflammatory diseases and disorders or as a thromboxane synthase inhibitor.

Throughout this specification and the claims which follow, unless the context requires otherwise, the word "comprise", and variations such as "comprises" or "comprising", will be understood to imply the inclusion of a stated integer or step or group of integers or steps but not the exclusion of any other integer or step or group of integers or steps.

### Brief Description of the Figures

Fig. 1 shows the effect of 10µM test compound on PGE₂ synthesis by human monocytes stimulated with LPS.
Fig. 2 shows the effect of 10µM test compound on TXB₂ synthesis by human monocytes stimulated with LPS.
Fig. 3 shows the effect of test compounds on TXB₂ synthesis following the addition of exogenous PGH₂ to U937 cells.
Fig. 4 shows the percent inhibition of NFκB promoter activity in TNFα-stimulated THP-1 monocyte/macrophages,
Fig. 5 shows the effect on colonic inflammation of Compounds 1 and 4 dosed orally at 1mg/kg (mean ± SEM).
Fig. 6 shows the effect on colonic length of Compounds 1 and 4 dosed orally at 1mg/kg (mean ± SEM).
Fig. 7 shows the effect on clinical score of Compounds 1 and 4 dosed orally at 1mg/kg.
Fig. 8 shows the effect on body weight of Compounds 1 and 4 dosed orally at 1mg/kg.
Fig. 9 shows the effect on colonic inflammation of Compounds 1 and 2 pre-dosed orally at 1mg/kg (mean ± SEM).
Fig. 10 shows the effect on the length of the colon of Compounds 1 and 2 pre-dosed orally at 1mg/kg (mean ± SEM).
Fig. 11 shows the effect on the clinical score of Compounds 1 and 2 pre-dosed orally at 1mg/kg.
Fig. 12 shows the effect on the body weight from the induction of colitis of pre-dosing Compounds 1 and 2 orally at 1mg/kg for 10 days.
Fig. 13 shows the effect on colonic inflammation of Compound 3a and 3b dosed orally at 1.25mg/kg (mean ± SEM).
Fig. 14 shows the effect on colonic length of Compound 3a and 3b dosed orally at 1.25mg/kg (mean ± SEM).
Fig. 15 shows the effect on clinical score of Compound 3a and 3b dosed orally at 1.25mg/kg.
Fig. 16 shows the effect on body weight of Compound 3a and 3b dosed orally at 1.25mg/kg.
Fig. 17 shows the production of PGE₂ and TXB₂ by colons cultured in the absence of mitogenic stimulation.
Fig. 18 shows the production of PGE2 and TXB2 by colons cultured in the presence of ConA.
Fig. 19 shows the percentage inhibition of the contractile repsonse of noradrenaline.
Fig. 20 shows the body weight change in rats given high doses of Compound 1 orally for 11 days.
Fig. 21 shows the body weight change following oral administration of Compound 2 at 5mg/kg/day or vehicle.
Fig. 22 shows the body weight change following oral administration of a mixture of Compounds 3a and 3b at 20mg/kg/day (data sets from individual mice and mean of group outlined in bold).

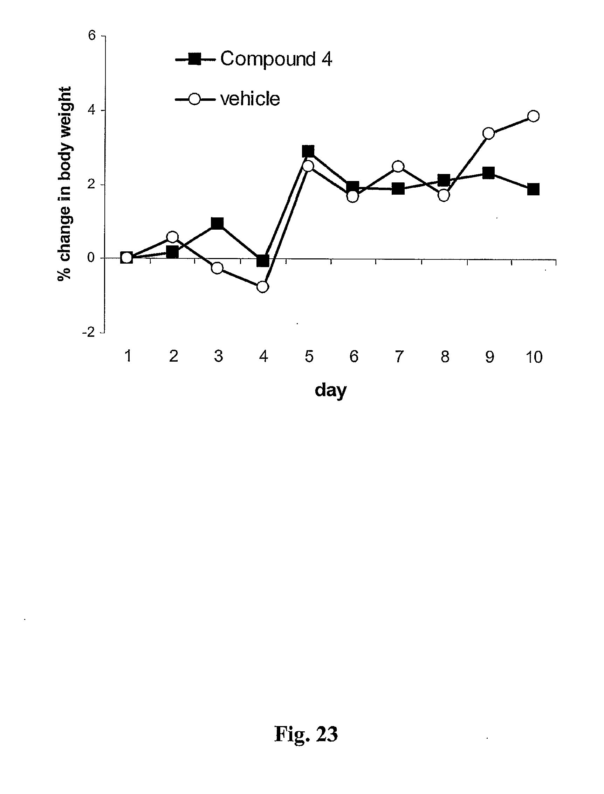

Fig. 23 shows the body weight change following oral administration of Compound 4 at 5mg/kg/day or vehicle.

### Detailed Description

The term alkyl is taken to include straight chain and branched chain saturated alkyl groups of 1 to 6 carbon atoms, such as methyl, ethyl, propyl, isopropyl, butyl, isobutyl, sec-butyl, tertiary butyl, pentyl and the like. The alkyl group more preferably contains from 1 to 4 carbon atoms, especially methyl, ethyl, propyl or isopropyl.

The compounds of the invention include all salts, such as acid addition salts, anionic salts and zwitterionic salts, and in particular include pharmaceutically acceptable salts as would be known to those skilled in the art. The term "pharmaceutically acceptable salt" refers to an organic or inorganic moiety that carries a charge and that can be administered in association with a pharmaceutical agent, for example, as a counter-cation or counter-anion in a salt. Pharmaceutically acceptable cations are known to those of skilled in the art, and include but are not limited to sodium, potassium, calcium, zinc and quaternary amine. Pharmaceutically acceptable anions are known to those of skill in the art, and include but are not limited to chloride, acetate, tosylate, citrate, bicarbonate and carbonate.

Pharmaceutically acceptable salts include those formed from: acetic, ascorbic, aspartic, benzoic, benzenesulphonic, citric, cinnamic, ethanesulphonic, fumaric, glutamic, glutaric, gluconic, hydrochloric, hydrobromic, lactic, maleic, malic, methanesulphonic, naphthoic, hydroxynaphthoic, naphthalenesulphonic, naphthalenedisulphonic, naphthaleneacrylic, oleic, oxalic, oxaloacetic, phosphoric, pyruvic, para-toluenesulphonic, tartaric, trifluoroacetic, triphenylacetic, tricarballylic, salicylic, sulphuric, sulphamic, sulphanilic and succinic acid.

The term "pharmaceutically acceptable derivative" or "prodrug" refers to a derivative of the active compound that upon administration to the recipient is capable of providing directly or indirectly, the parent compound or metabolite, or that exhibits activity itself and includes for example phosphate derivatives and sulphonate derivatives. Thus, derivatives include solvates, pharmaceutically active esters, prodrugs or the like.

The preferred compounds of the present invention also include all derivatives with physiologically cleavable leaving groups that can be cleaved *in vivo* to provide the compounds of the invention or their active moiety. The leaving groups may include acyl, phosphate, sulfate, sulfonate, and preferably are mono-, di- and per-acyl oxy-substituted compounds, where one or more of the pendant hydroxy groups are protected by an acyl group, preferably an acetyl group. Typically acyloxy substituted compounds of the invention are readily cleavable to the corresponding hydroxy substituted compounds.

The invention also provides a pharmaceutical composition comprising a compound of formula (I) and at least one pharmaceutically acceptable excipient, especially for use in treatment or in the manufacture of a medicament, for example, for the treatment of inflammatory bowel disease (IBD), for example: ulcerative colitis (UC), ulcerative proctitis, distal colitis, and/or Crohn's disease (CD), as well as other intestinal syndromes including primary sclerosing cholangitis (PSC), primary biliary cirrhosis (PBC), autoimmune hepatitis (AIH) and irritable bowel syndrome (IBS).

In the context of this application substantially pure is intended to mean 90% purity or greater such as 95% purity, particularly 98% purity, especially 99% purity, for example as assessed by HPLC analysis.

The invention also extends to employing at least two compounds of formula (I) in the various aspects of the invention described herein.

Pharmaceutical formulations include those suitable for oral, parenteral (including subcutaneous, intradermal, intramuscular, intravenous and intraarticular), inhalation (including use of metered dose pressurised aerosols, nebulisers or insufflators), rectal and topical (including dermal, buccal, sublingual and intraocular) administration. The most suitable route may depend upon for example the condition and disorder of the recipient. The formulations may conveniently be presented in unit dosage form and may be prepared by any of the methods well known in the art of pharmacy. All methods include the step of bringing the active ingredient into association with the carrier which constitutes one or more accessory ingredients. In general the formulations are prepared by uniformly and intimately bringing into association the active ingredient with liquid carrier or finely divided solid carriers or both and then, if necessary, shaping the product into the desired formulation.

Formulations of the present invention suitable for oral administration may be presented as discrete units such as capsules such as gelatine or HPMC capsules, cachets or tablets each containing a predetermined amount of the active ingredient; as a powder or granules; as a solution or a suspension in an aqueous liquid or a non-aqueous liquid; or as an oil-in-water liquid emulsion or a water-in-oil liquid emulsion. The active ingredient may also be presented as a paste.

When compounds of formula (I) are formulated as capsules preferably the compound is formulated with one or more pharmaceutically acceptable carrier such as starch, lactose, microcrystalline cellulose, silicon dioxide and/or a cyclic oligosaccaride such as cyclodextrin. Additional ingredients may include lubricants such as magnesium stearate and/or calcium stearate.

Suitable cyclodextrins include α-cyclodextrin, β-cyclodextrin, γ-cyclodextrin, demethyl-β-cyclodextrin, 2-hydroxyethyl-β-cyclodextrin, 2-hyroxypropyl-cyclodextrin, 3-hydroxypropyl-β-cyclodestrin and tri-methyl-β-cyclodextrin. More preferably the cyclodextrin is hydroxypropyl-β-cyclodextrin.

Tablets may be prepared by compression or moulding, optionally with one or more accessory ingredients. Compressed tablets may be prepared by compressing in a suitable machine the active ingredient in a free-flowing form such as a powder or granules, optionally mixed with a binder, lubricant such as magnesium stearate or calcium stearate, inert diluent or a surface active/dispersing agent. Moulded tablets may be made by moulding a mixture of the powdered compound moistened with an inert liquid diluent, in a suitable machine. The tablets may optionally be coated, for example, with an enteric coating and may be formulated so as to provide slow or controlled release of the active ingredient therein.

Formulations for parenteral administration include aqueous and non-aqueous sterile injection solutions which may contain anti-oxidants, buffers, bacteriostats and solutes which render the formulation isotonic with the blood of the intended recipient and which may include suspending agents and thickening agents. Preferably a parenteral formulation will comprise a cyclic oligosaccaride such as hydroxypropyl-β-cyclodextrin. The formulations may be presented in unit-dose or multi-dose containers, for example sealed ampoules and vials, and may be stored in a freeze-dried (lyophilised) condition requiring only the addition of the sterile liquid carrier, for example saline or water-for-injection, immediately prior to use. Extemporaneous injection solutions and suspensions may be prepared from sterile powders, granules and tablets of the kind previously described.

Dry powder compositions for topical delivery to the lung by inhalation may, for example, be presented in capsules and cartridges of for example gelatine, or blisters of for example laminated aluminium foil, for use in an inhaler or insufflator. Formulations generally contain a powder mix for inhalation of the one or more compounds of the invention and a suitable powder base (carrier substance) such as lactose or starch. Use of lactose is preferred. Each capsule or cartridge may generally contain between 20µg-10mg of the compound formula (I) optionally in combination with another therapeutically active ingredient. Alternatively, the compound or compounds of the invention may be presented without excipients. Packaging of the formulation may be for unit dose or multi-dose delivery.

Spray compositions for topical delivery to the lung by inhalation may, for example be formulated as aqueous solutions or suspensions or as aerosols suspensions or solutions delivered from pressurised packs, such as a metered dose inhaler, with the use of a suitable liquefied propellant. Suitable propellants include a fluorocarbon or a hydrogen-containing chlorofluorocarbon or mixtures thereof, particularly hydrofluoroalkanes, e.g. dichlorodifluoromethane, trichlorofluoromethane, dichlorotetra-fluoroethane, especially 1,1,1,2-tetrafluoroethane, 1,1,1,2,3,3,3-heptafluoro-n-propane or a mixture thereof. Carbon dioxide or other suitable gas may also be used as propellant. The aerosol composition may be excipient free or may optionally contain additional formulation excipients well known in the art such as surfactants eg oleic acid or lecithin and cosolvents eg ethanol. Pressurised formulations will generally be retained in a canister (eg an aluminium canister) closed with a valve (eg a metering valve) and fitted into an actuator provided with a mouthpiece.

Medicaments for administration by inhalation desirably have a controlled particle size. The optimum particle size for inhalation into the bronchial system is usually 1-10µm, preferably 2-5µm. Particles having a size above 20µm are generally too large when inhaled to reach the small airways. When the excipient is lactose it will typically be present as milled lactose, wherein not more than 85% of lactose particles will have a MMD of 60-90µm and not less than 15% will have a MMD of less than 15µm.

Formulations for rectal administration may be presented as a suppository with carriers such as cocoa butter or polyethylene glycol, or as an enema wherein the carrier is an isotonic liquid such as saline. Additional components of the formulation may include a cyclic oligosaccaride, for example, a cyclodextrin, as described above, such as hydroxypropyl-β-cyclodextrin, one or more surfactants, buffer salts or acid or alkali to adjust the pH, isotonicity adjusting agents and/or anti-oxidants.

It should be understood that in addition to the ingredients particularly mentioned above, the formulations of this invention may include other agents conventional in the art having regard to the type of formulation in question, for example those suitable for oral administration may include flavouring agents.

The compounds and pharmaceutical formulations according to the invention may be used in combination with or include one or more other therapeutic agents, for example anti-inflammatory agents, anticholinergic agents (particularly an M₁, M₂, M₁/M₂ or M₃ receptor antagonist), β₂-adrenoreceptor agonists, antiinfective agents (e.g. antibiotics, antivirals), or antihistamines. Preferred are combinations comprising a compound or compounds of formula (I) or a pharmaceutically acceptable salt, solvate or physiologically functional derivative thereof together with a corticosteroid, and/or an anticholinergic, and/or a PDE-4 inhibitor.

Suitable anti-inflammatory agents include corticosteroids and NSAIDs. Suitable corticosteroids, which may be used in combination with the compounds of the invention are those oral and inhaled corticosteroids and their pro-drugs which have anti-inflammatory activity. Examples include methyl prednisolone, prednisolone, dexamethasone, fluticasone propionate, 6α,9α-difluoro-17α-[(2-furanylcarbonyl)oxy]-11β-hydroxy-16α-methyl-3-oxo-androsta-1,4-diene-17β-carbothioic acid *S*-fluoromethyl ester, 6α,9α-difluoro-11β-hydroxy-16α-methyl-3-oxo-17α-propionyloxy-androsta-1,4-diene-17β-carbothioic acid *S*-(2-oxo-tetrahydro-furan-3S-yl) ester, beclomethasone esters (e.g. the 17-propionate ester or the 17,21-dipropionate ester), budesonide, flunisolide, mometasone esters (e.g. the furoate ester), triamcinolone acetonide, rofleponide, ciclesonide and butixocort propionate. Preferred corticosteroids include fluticasone propionate, and 6α,9α-difluoro-17α-[(2-furanylcarbonyl)oxy]-11 β-hydroxy-16α-methyl-3-oxo-androsta-1,4-diene-17β-carbothioic acid *S*-fluoromethyl ester, more preferably 6α,9α-difluoro-17α-[(2-furanylcarbonyl)oxy]-11β-hydroxy-16α-methyl-3-oxo-androsta-1,4-diene-17β-carbothioic acid *S*-fluoromethyl ester.

Suitable NSAIDs include sodium cromoglycate, nedocromil sodium, phosphodiesterase (PDE) inhibitors (e.g. theophylline, PDE4 inhibitors or mixed PDE3/PDE4 inhibitors), leukotriene antagonists, inhibitors of leukotriene synthesis, iNOS inhibitors, tryptase and elastase inhibitors, beta-2 integrin antogonists and adenosine receptor agonists or antagonists (e.g. adenosine 2a agonists), cytokine antagonists (e.g. chemokine antagonists) or inhibitors or cytokine synthesis.

The co-administration of active ingredients may be simultaneous or sequential. Simultaneous administration may be effected by the compounds being in the same unit dose, or in individual and discrete unit doses administered at the same or similar time. Sequential administration may be in any order as required and typically will require an ongoing physiological effect of the first or initial active agent to be current when the second or later active agent is administered, especially where a cumulative or synergistic effect is desired.

Preferably the formulation is an oral formulation, more preferably a capsule formulation.

Preferably the capsule formulation will comprise consist essentially of or consist of a compound of formula (I) and silicon dioxide.

Preferably the capsule will be a HPMC capsule.

In an alternatively embodiment the formulation is a suppository or enema, which can be used to direct the active ingredient more closely to the disease affected area of the body.

Preferably compositions according to the present invention, oral or otherwise, will comprise 5mg per kg or less, for example, 0.01 to 4mg per kg such as 0.5 to 3mg per kg of the body weight of the patient of compounds of formula (I).

For example, formulations may comprise 50 to 500mg of compounds of formula (I) per unit dose, more preferably 200 to 400mg such as 250mg.

Thus the invention provides one or more compounds of formula (I) and/or compositions comprising the same for use in treatment or the manufacture of a medicament.

The invention also provides a methods of treatment comprising administering to a patient in need thereof a therapeutically effective amount of one or more compounds of formula (I) or a composition comprising same. These methods are particularly effective for the diseases listed above.

Preferably the compounds and compositions according to the invention will be employed in the treatment of IBD, for example, colitis such as ulcerative colitis, ulcerative proctitis, distal colitis; Crohn's disease and/or the prevention of colon cancer.

In another preferred aspect the compounds and compositions of the present invention are useful in the maintenance of remission of said diseases, especially IBD.

Ultimately, the dose prescribed and intervals at which the prescribed dose is taken will be at the discretion of the physician with responsibility for the patient.

Compounds of formula (I) may be administered, for example, at doses in the range of 50 to 500mg, for example, 1 to 4 times daily such as once or twice daily.

The compounds and compositions comprising the same have surprisingly low toxicity. Furthermore, they are advantageous in that they are more selective, less toxic and/or more efficacious, for example, in reducing clinical symptoms such as weight loss, gastrointestinal ulceration and the like, than compounds of a similar structure.

Whilst not wishing to be bound by theory it is thought that the compounds of the present invention are selective thromboxane synthase inhibitors. Thromboxanes (TX) may play a major pathogenic role in IBD. TXs are produced in excess not only in inflamed gut mucosa but also by isolated intestinal and peripheral blood mononuclear cells and uninflamed bowel in Crohn's disease.

These isoflavan-4-ones and isoflavan-4-ols and derivatives thereof are readily obtainable by standard procedures known in the art. Published International patent applications WO 98/08503, WO00/49009 and WO 01/17986 (all to Novogen Research Pty Ltd), and references cited therein, which disclosures are incorporated herein by reference, also provide useful synthetic methods for the production of isoflavones, isoflavanones, isoflavan-4-ols and related isoflavonoid compounds as the starting monomers. A representative general synthesis is set out in Scheme 1 below.

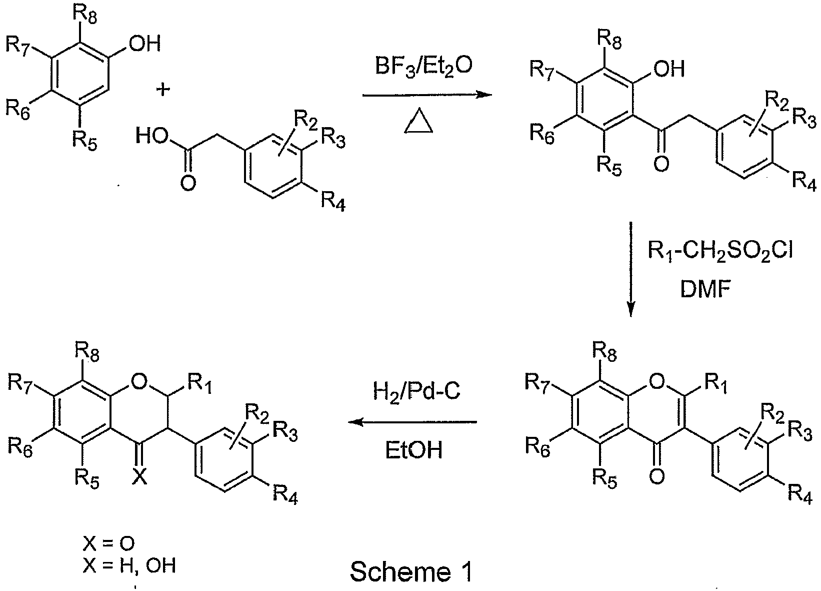

Access to various substitution patterns around both the benzopyran ring and the pendant phenyl ring of the isflavan-4-ols is made possible by selecting correspondingly substituted R5-R8-phenols and R2-R4-phenyl acetic acid starting materials. The ring cyclisation reactions may conveniently be preformed with R1-substituted methanesulfonyl chloride reactants as would be known to those skilled in the art. The reduction reaction is successfully performed with Pd-C or Pd-alumina in an alcoholic solvent in the presence of hydrogen. The conditions can dictate the end product of reduction. The person skilled in the art will be aware that other standard methods of alkylation, cyclisation, hydrogenation and/or reduction can be employed as appropriate.

The protection of functional groups on the compounds and derivatives of the present invention can be carried out by well established methods in the art, for example as described in T. W. Greene, Protective Groups in Organic Synthesis, John Wiley & Sons, New York, 1981.

Hydroxyl protecting groups include but are not limited to carboxylic acid esters, eg acetate esters, aryl esters such as benzoate, acetals/ketals such as acetonide and benzylidene, ethers such as *ortho*-benzyl and *para*-methoxy benzyl ether, tetrahydropyranyl ether and silyl ethers such as *tert*-butyldimethyl silyl ether.

Protecting groups can be removed by, for example, acid or base catalysed hydrolysis or reduction, for example, hydrogenation. Silyl ethers may require hydrogen fluoride or tetrabutylammonium fluoride to be cleaved.

Specifically, compound of formula (I) can be prepared, for example, by the following methods. Herein the substitution pattern is of a preferred embodiment, being the 8-substituted compounds, however it will be understood that by varying the starting materials different substitution patterns may be readily obtained.

Isoflavanones and isoflavan-4-ols are readily obtainable by the selective reduction of isoflavones (see Novogen WO00/49009). Reducing agents are well known to persons skilled in the art and can include hydride sources like borohydrides and alkali metal borohydrides, but would include hydrogen in catalytic hydrogenation where a suitable catalyst such as palladium on carbon or platinum oxide is employed. Other suitable hydride sources include sodium triacetoxyborohydride and sodium cyanoborohydride.

Preferably the double bond is reduced by hydrogenation. Preferably the catalyst employed is palladium on carbon or platinum oxide.

Resorcinol is the key starting material in Scheme 1 above. Substituted resorcinols are readily available or can be synthesised as required. For example, resorcinol may be methylated or halogenated by treating resorcinol with a halogenating agent, for example in the presence of a suitable solvent. Suitable halogenating agents include iodine, bromine, chlorine, or fluorine. It may be necessary to perform the halogenation step at a reduced temperature, for example 0°C.

The compounds of formuala (I) and all intermediate compounds also form aspects of the invention, as do processes for the preparation of all the compounds described herein.

The compound 3,4-epoxy-4',7-dihydroxyisoflavan also shows anti inflammatory activity. It is obtained by dehydration (P₂O₅) of 4',7-dihydroxyisoflavan-4-ol to 4',7-dihydroxyisoflav-3-ene. Epoxidation (*m*-CPBA) of the double bond affords the epoxide.

The inventors have found that inhibition of the eicosanoid thromboxane (TX), by either selectively or non-selectively inhibiting thromboxane synthase (TXS), has the pleiotropic functions of:
1. anti-inflammatory activity, manifest by a reduction in the classical clinical signs of pain, erythema and swelling, as well as
2. cardioprotective activity, either by reducing hypertension, modifying serum lipids and/or by having anti-atherosclerotic activity, or by eliminating or reducing the prothrombotic effects of COX-2 inhibition, which contribute to increased cardiovascular risk.
3. gut protective effects via either increased production or reduced inhibition of prostaglandins, thus avoiding the side effects of NSAIDS

### Anti-inflammatory activity

Prostaglandins eg PGE₂ and PGI₂ and thromboxanes (TXs) eg TXA₂ are members of a family of fatty acid derivatives known as eicosanoids (Penglis et al. 2000). They are involved in both normal physiology and inflammatory responses, but have opposing effects on eg cytokine release and platelet aggregation. Release of arachidonic acid (AA) from membrane phospholipids provides the primary substrate for eicosanoid synthesis. Action of the cyclooxygenase (COX) enzymes, regardless of isotype, causes synthesis of the intermediate prostaglandin PGH₂, the common precursor for PGE₂, PGI₂ and TXA₂.

The inventors have found that inhibition of thromboxane itself is an anti-inflammatory strategy.
1. Prostanoids play an important modulatory role in the immune response through complex interactions with leucocytes and parenchymal cells in the inflamed organ. They can produce both pro- and anti-inflammatory actions depending upon the inflammatory stimulus, the predominant prostanoid produced, and the profile of prostanoid receptor expression (Tilley et al. 2001).
2. The thromboxane TXA₂ is a prostanoid with actions that appear to be primarily pro-inflammatory (Thomas et al. 2003). Enhanced production of TX has been implicated in the pathogenesis of various immune-mediated diseases eg lupus nephritis.
3. The inventors have demonstrated that the isoflavonoid compounds of the invention and which are TXS inhibitors have marked anti-inflammatory activity according to an novel and selective mode of action in a variety of animal models.
4. Thromboxanes play a major pathogenic role in inflammatory bowel disease (IBD). TXs are produced in excess not only in inflamed mucosa but also in Crohn's disease by uninflamed bowel and by isolated intestinal and peripheral blood mononuclear cells. Their cellular source is likely to include platelets, neutrophils, endothelial and epithelial cells as well as mononuclear cells (Hawkey et al. 1985; Rampton and Collins 1993; McCartney et al. 1999; Carty et al. 2000; Carty et al. 2002). The pro-inflammatory effects of TXs are both direct (diapedesis and activation of neutrophils, mucosal ulceration, reduction of suppressor T-cell activity) and indirect (vasoconstriction, platelet activation). PGs are thought to be protective to gastrointestinal mucosa (Carty et al. 2000). Sulfasalazine, a compound frequently administered in the treatment of chronic IBD, as well as one of its main metabolites, sulfapyridine, have been demonstrated to inhibit synthesis of TXB₂ while enhancing synthesis of PGF_{2α} or PGE₂, respectively (Hawkey et al. 1985). In other words, they would appear to have some level of TX synthase inhibition
5. Inhibition of TX synthase leads to reduced formation of TXs, and because there is an increased availability of the substrate PGH₂ for PG synthase, an increase in synthesis of PGs, (Carty et al. 2000; Penglis et al. 2000). An increase in PGE₂ can exert anti-inflammatory effects. For example:
   a. PGE₂ has been reported to attenuate some acute inflammatory responses, in particular those initiated by mast cell degranulation (Raud et al. 1988).
   b. PGE₂ suppresses, whereas TXA₂ increases TNF_{α} and IL-1β (Caughey et al. 1997). Inhibition of TXA₂ is a potential way of inhibiting inflammatory cytokine production, particularly that of TNF. Currently, biological therapies which suppress TNF levels (with antibodies or soluble TNF receptor shave been successful in treating rheumatoid arthritis which is refractory to, or no longer responsive to other therapies. A chemical agent which suppressed TNF production and which could be taken orally would be a great advance. Inhibition of TXA₂ formation may be a means of suppressing production of TNF, a cytokine which is involved in the signs and symptoms of joint inflammation and in the longer term degradative phase of joint inflammation manifest in cartilage degradation, diminution of joint space and ultimately, joint failure.
   c. PGE₂ inhibits a wide range of T and B cell functions including inhibition of T lymphocyte activation and proliferation and Ig production (Tilley et al. 2001). Conversely, TXA₂ may promote T cell activation and proliferation and facilitate the development of effector cytolytic T cells (CTLs). Altering this balance in favour of PG production may facilitate 'quenching' of an inappropriate immune response as occurs in autoimmune disease.
   d. In asthma, PGE₂ promotes vasodilation and increases vascular permeability (Tilley et al. 2001). As inflammation progresses, PGE₂ synthesis by macrophages is enhanced due to increased expression of COX-2 and PGE-synthase. PGE₂ inhibits leukocyte activation and promotes bronchodilation. TXA₂ synthase inhibitors and thromboxane prostanoid (TP) receptor antagonists have been developed as antiasthma drugs (Shi et al. 1998).
   e. In glomerulonephritis there is co-activation of the AA COX pathway toward synthesis of PGs and TX and of lipoxygenase pathways toward synthesis of leukotrienes. TXA₂ is the most abundant eicosanoid synthesized in nephritic glomeruli, and TXA₂ synthase inhibitors (eg Dazmegrel) are now available for the treatment of glomerulonephritis. In a rat model of nephritis, Dazmegrel increased PGE₂ synthesis which is useful as PGE₂ preserves kidney function in glomerulonephritis (Lianos and Bresnahan 1999).
   f. COX-2-derived prostaglandins are associated with resolution of inflammation. The profile of PGs being produced in the inflammatory lesion changes from pro-inflammatory, PGE₂-dominated during the development of inflammation to anti-inflammatory, cyclopentenone (cyPG)-dominated during the resolution of inflammation (Gilroy et al. 1999).

### Cardioprotective activity

An inhibition of thromboxane itself is a cardioprotective strategy, in contrast to other anti-inflammatory agents which utilise COX inhibition.
1. The ratio of PG to TX is dependent on which isoform of COX (COX-1 or COX-2) is present (Caughey et al. 2001). TXA₂ is the major COX-1-derived product, but the induction of COX-2 results in a preferential increase in PGE₂ and PGI₂ synthesis. Conversely, when COX-2 is inhibited, there is an increase in TX synthesis.
2. TXA₂ is a potent inducer of platelet aggregation. The increased cardiovascular risk in the rofecoxib (Vioxx) case was due to the pro-thrombotic consequence of an increase in TX synthesis caused by selective COX-2 inhibition. Non-selective COX inhibition also causes some cardiovascular risk, because the substrate for both TX and PGs, PGH₂ is inhibited by either or both COX-1 or -2.
3. Therefore, if the inhibition of COX is removed from the anti-inflammatory 'equation' entirely, as would happen with specific TXS inhibition, an inflammatory mediator ie TXA₂ is inhibited yet there is no increase in cardiovascular risk.
4. TXA₂ is also vasoconstrictive, so its inhibition is likely to contribute to these compounds being anti-hypertensive. There is a link between angiotensin II and TXA₂ in cardiovascular function (Dogne et al. 2004). For example, TXA₂ and PGH₂ are involved in angiotensin II-dependent hypertension by stimulating the contraction of vascular smooth muscle. Moreover, some commonly used angiotensin 11-inhibitors are also thromboxane receptor (TP) antagonists which inhibit platelet aggregation.

### Gastrointestinal protective activity

Because inhibition of TXS causes an increased availability of the PG substrate, PGH₂, it is possible that the synthesis of PGE₂ would be increased, or if not increased, at least not be inhibited to the extent that occurs with the inhibition of COX. The use of NSAIDs, which inhibit COX, is associated with gastrointestinal ulceration, perforation and bleeding, due to the inhibition of PGE₂.

Whilst PGs are included in the list of eicosanoids which mediate the hallmarks of inflammation - pain, swelling and erythema - they also have a beneficial role in protecting gastric mucosa from the hydrochloric acid it produces (Vane and Botting 2003). This occurs in a variety of ways:
1. Exogenously administered PGs can prevent disruption of the gastric mucosal barrier, enhance gastric mucosal blood flow and stimulate mucus and bicarbonate secretion (Miller 1983).
2. Inhibition of PGE₂ reduces proliferation of gastric mucosal cells *in vivo* (Levi et al. 1990). Consequently, it would follow that PGE₂ is important in the maintenance of mucosal integrity.
3. Endogenous PGs play a central role in adaptive cytoprotection induced in the stomach by mild irritants (Robert et al. 1979). Pre-treatment of cultured gastric mucous cells with PGE₂ reduced ethanol-caused injury of the cells (Sakamoto et al. 1993). Furthermore, pre-treatment of gastric mucous cells with the non-selective COX inhibitor indomethacin enhanced ethanol-caused injury, suggesting that endogenous PGE₂ is involved in the cell protection. The receptors activated by PGE₂ are pharmacologically subdivided into at least four subtypes (EP₁, EP₂, EP₃, and EP₄) and *in vivo,* adaptive gastric cytoprotection is mediated by activation of EP₁-receptors (Takeuchi et al. 2001).
4. The protective role of PGE₂ also involves modulation of the gastric mucosal microcirculation. Disruption of the balance between the local release of vasodilator eg PGE₂ and vasoconstrictor mediators eg endothelium-derived peptide endothelin-1 (ET-1) is thought to be involved in the pathogenesis of mucosal injury (Whittle and Lopez-Belmonte 1993).

PGE₂ reduces radiation-induced apoptosis in the mouse small intestine and endogenous PGE₂ in the intestine increases epithelial crypt survival after radiation (Cohn et al. 1997). This effect is mediated by signalling through EP₂, the phosphorylation of AKT, and the inhibition of *bax* translocation (Tessner et al. 2004).

It is also surprising the compounds of the present invention are thought to be chemo-sentising agents, that is they increase the cytotoxicity of the one or more anticancer drugs co-administered therewith, and/or radio-sensitising agents by which it is meant that these compounds either lower the amount of gamma-irradiation that is required to kill cancerous cells, or they convert cancer cells from a state of radio-resistance to radio-sensitivity.

In addition, the compounds of the present invention are useful in the treatment and prevention of a range of important human disease including cancers, inflammatory disorders, autoimmune disorders, cardiovascular disorders, and disorders associated with estrogen receptor activation.

The invention is further illustrated by the following non-limiting Examples and accompanying drawings.

### Examples

### Synthetic Methods for substituted isoflavanones and isoflavan-4-ols

### Condensation of 2-bromoresorcinol with 4-hydroxyphenylacetic acid - Synthesis of 1-(3-bromo-2,4-dihydroxyphenyl)-2-(4-hydroxyphenyl)-ethanone

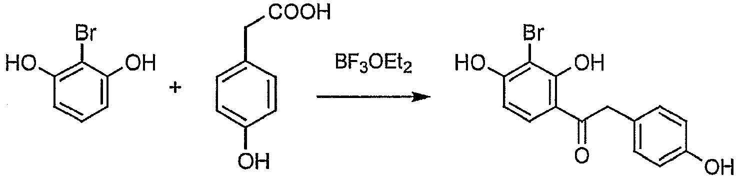

Borontrifluoride diethyl etherate (30ml) was added to a mixture of 2-bromoresorcinol (5.0 g, 26 mmoles) and 4-hydroxyphenylacetic acid (3.0g, 26 mmoles) in a round bottom flask fitted with a condenser and a magnetic stirrer. The mixture was heated at 100-110°C for one and a half hour with stirring. The mixture was then cooled to room temperature and kept in a freezer overnight. The precipitated solid was filtered and recrystallised from ethanol to give deoxybenzoin as off-white crystals. The crystals were then collected and dried in a desiccator. ¹H NMR (CDCl₃): δ 4.16 (s, 2H, CH₂), 6.61 (d, 1H, J 9.0 Hz, ArH), 6.80 (d, 2H, J 8.6 Hz, ArH), 7.12 (d, 1H, J 8.6 Hz, ArH), 7.75 (d, 1H, J 9.0 Hz, ArH).
Yield: 5.2g, 69%

### Cyclisation of 1-(3-bromo-2,4-dihydroxyphehyl)-2-(4-hydroxyphenyl)-ethanone - Synthesis of 3-(4-hydroxyphenyl)-7-hydroxy-8-bromo-chromen-4-one

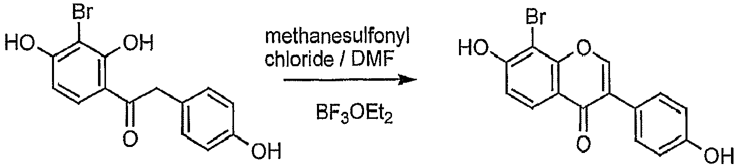

Boron trifluoride diethyl etherate (6 ml) was added dropwise to a solution of 1-(3-bromo-2,4-dihydroxyphenyl)-2-(4-hydroxyphenyl)-ethanone (2.5 g) in dry dimethyformamide (20 ml) maintained at 50°C. Methanesulfonyl chloride (3.3 ml) was then added to the solution and the mixture heated at 110°C for 1 hour. After cooling to room temperature, hydrochloric acid (2M, 120 ml) was slowly added to the reaction mixture. The resulting yellow precipitate was filtered, washed thoroughly with water and dried.
¹H NMR (d₆-DMSO): δ 6.79 (d, 2H, J 8.7 Hz, ArH),7.08 (d, 1H, J 8.7 Hz, H6), 7.36 (d, 2H, J 8.7 Hz, ArH), 7.94 (d, 1H, J 8.7 Hz, H5), 8.39 (s, 1H, H2).
Yield 2.35g

The crude product was used in the next step without any further purification.

### Acetylation of 3-(4-hydroxyphenyl)-7-hydroxy-8-bromo-chromen-4-one - Synthesis of 3-(4-acetoxyphenyl)-7-acetoxy-8-bromo-chromen-4-one

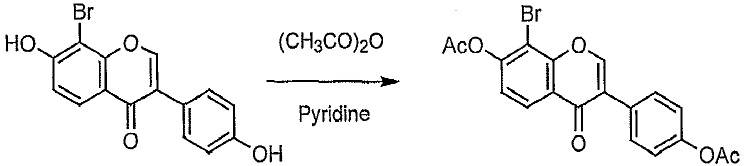

Acetic anhydride (15 ml) and pyridine (2.5 ml) were added to 3-(4-hydroxyphenyl)-7-hydroxy-8-bromo-chromen-4-one (2.35g) in a round bottom flask fitted with a condenser and magnetic stirrer. The mixture was heated to 110°C for 1 hour. It was then cooled and left in a refrigerator to crystallise. The white crystalline solid was filtered and washed several times with water. Recrystallisation of the crude product from ethanol afforded 3-(4-acetoxyphenyl)-7-acetoxy-8-bromo-chromen-4-one as white crystalline needles.
¹H NMR (CDCl₃): δ 2.32, 2.42 (each s, 3H, OCOCH₃), 7.17-7.22 (m, 3H, ArH),7.58 (d, 2H, J 8.7 Hz, ArH), 8.1 (s, 1H, H2), 8.29 (d, 1H, J 9.0 Hz, H5).
Yield: 1.5g

### Hydrogenation of 3-(4-acetoxyphenyl)-7-acetoxy-8-bromo-chromen-4-one - Synthesis of 3-(4-acetoxyphenyl)-7-acetoxy-4-hydroxy-8-bromo-chroman-4-one and 3-(4-acetoxyphenyl)-7-acetoxy-4-hydroxy-8-bromo-chroman-4-ol

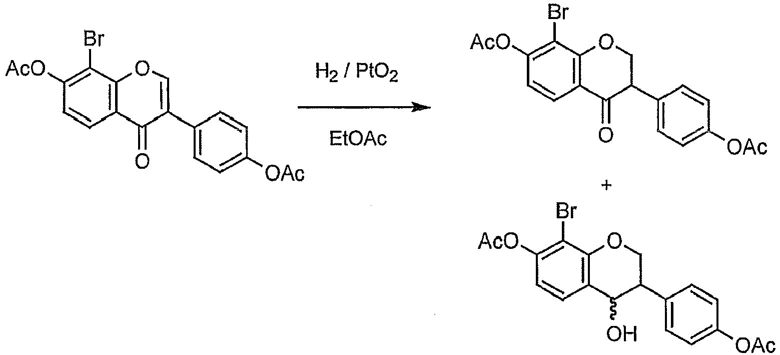

A 250 ml round-bottom flask containing a suspension of 3-(4-acetoxyphenyl)-7-acetoxy-8-bromo-chromen-4-one (0.3 g) in ethyl acetate (80 ml) was evacuated and filled with argon to provide an inert atmosphere. Platinum oxide (67 mg) was rapidly added the mixture and the mixture was gently swirled to ensure that the catalyst is completely covered with ethyl acetate. The mixture was hydrogenated under standard conditions at room temperature for 3 days. The solution was filtered through a pad of Celite to remove the catalyst and the solution was evaporated in *vacuo* to yield a colourless oil. A tlc analysis of the oil indicated it to be mixture of chroman-4-one and chroman-4-ol. The crude product was chromatographed on a silica column using dichloromethane/ethyl acetate (98:2) as the eluent.

### 3 -(4-acetoxyphenyl)-7-acetoxy-4-hydroxy-8-bromo-chroman-4-ol Yield 100 mg

¹H NMR (CDCl₃): □ 2.30, 2.35 (each s, 3H, OCOCH₃), 3.32 (dt, 1H, J 3.4 Hz, J 11.7 Hz, H3), 4.48 (m, 1H, H2); 4.65 (dd, 1H, J 10.5 Hz, 11.7 Hz, H2), 4.78 (bs, 1H, H4), 6.75 (d, 1H, J 8.3 Hz, H6), 7.10 (d, 2H, J 8.3 Hz, ArH), 7.25 (d, 1H, J 8.3 Hz, H5), 7.30 (d, 2H, J 8.3 Hz, ArH).

### 3-(4-Acetoxyphenyl)-7-acetoxy-8-bromo-chroman-4-one Yield 170 mg

¹H NMR (CDCl₃): 2.29, 2.38 (each s, 3H, OCOCH₃), 4.02 (dd, 1H, J 5.7 and 8.7 Hz, H3), 4.60 (m, 2H, H2), 6.88 (d, 1H, J 8.6 Hz, H6),7.09 (d, 2H, J 8.7 Hz, ArH), 7.29 (d, 2H, J 8.7 Hz, H2), 7.96 (d, 1H, J 8.6 Hz, H5).

### 3-(4-hydroxyphenyl)-7-hydroxy-8-bromo-chroman-4-one

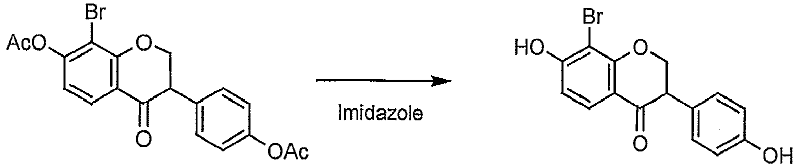

Imidazole (0.50g) was added to a suspension of 3-(4-acetoxyphenyl)-7-acetoxy-8-bromochroman-4-one (0.17) in absolute ethanol (7.0 ml) and the mixture was refluxed for 1h under argon. The solution was concentrated under reduced pressure and distilled water (5 ml) and hydrochloric acid (1M, 5 ml)was added to the residue. The mixture was left overnight in the fridge and the resulting creamy white solid was filtered, washed thoroughly with water and dried in a vacuum desiccator.
Yield 130 mg
¹H NMR(d₆-acetone): □ 3.93 (t, 1H, J 6.8 Hz, H3), 4.60 (d, 2H, J 7.2 Hz, H2), 6.75-6.81 (m, 3H, ArH), 7.13 (d, 2H, J 8.7 Hz, ArH), 7.72 (d, 1H, J 8.6 Hz, H5), 8.44 (bs, 1H, OH), 10.2 (bs, 1H, OH).

A similar deprotection of the acetoxy isoflavan-4-one leads to the corresponding hydroxy compound. By varying the substitution pattern of the starting resorcinol, the other compounds of the invention were synthesised. All compounds synthesised showed spectral data consistent with the assigned structures.

The synthesis of Compound 2 started with the condensation of pyrogallol with 4-hydroxyphenylacetic acid. Further reaction as above afforded compound 2. Compounds 3, 4 and 5 begin with 2-methylresorcinol, 4-chlororesorcinol and 5-methylresorcinol respectively.

### ANTI-INFLAMMATORY ACTIVITY

### INHIBITION OF EICOSANOIDS INDUCED IN HUMAN MONOCYTES

Human peripheral blood monocytes (from three separate individuals) were isolated from buffy coats by lymphoprep gradient separation of mononuclear cells followed by countercurrent centrifugal elutriation (Demasi et al. 2000). Test compounds were dissolved in DMSO and added to fresh monocytes to achieve concentrations of 0, 10 and 100µM. After 30min, lipopolysaccharide (LPS) was added to achieve a final concentration of 200ng/mL. After incubation for 18hrs at 37°C in 5% CO₂, supernatants were removed and PGE₂ and TXB₂ (the stable hydrolysis product of TXA₂) production were measured by radioimmunoassay (RIA). Except for Compound 4, which was only tested once, each compound was examined in three different assays. ANOVA followed by Newman-Keuls multiple comparisons test was used to examine differences between doses and the control values. A statistical significance level of 0.05 was used and differences from control values.

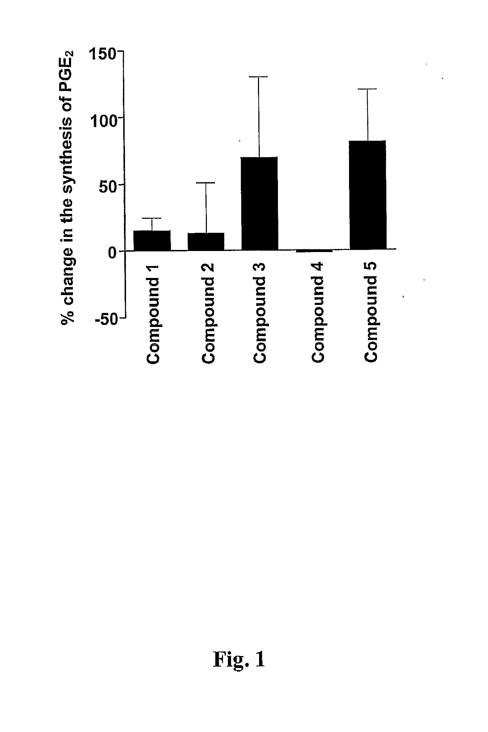

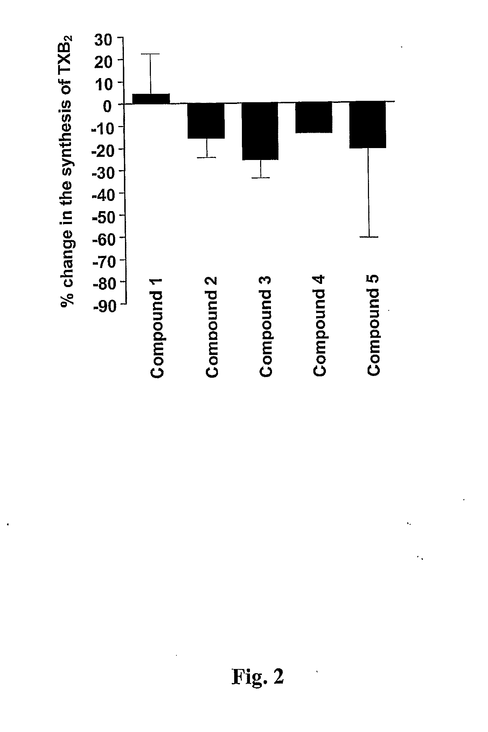

The results obtained at a concentration of 10µM are presented in Fig. 1 (PGE₂) and Fig. 2 (TXB₂). Because PGE₂ was either induced in a dose responsive manner by the test compounds or the effect on PGE₂ was minimal, and generally TXB₂ was inhibited, these results suggested that the compounds were thromboxane (TX) synthase inhibitors.

To examine effects of test compounds directly on TX synthase (TXS), a different assay system was used. Whereas arachidonic acid (AA) is the substrate for COX, PGH₂ is the substrate for TX synthase. To examine directly the effect of these compounds on TXS, exogenot

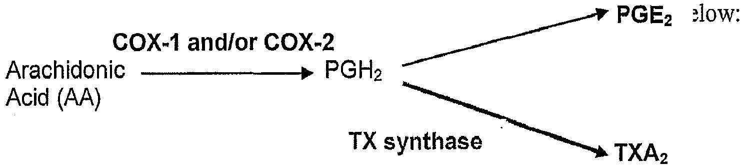

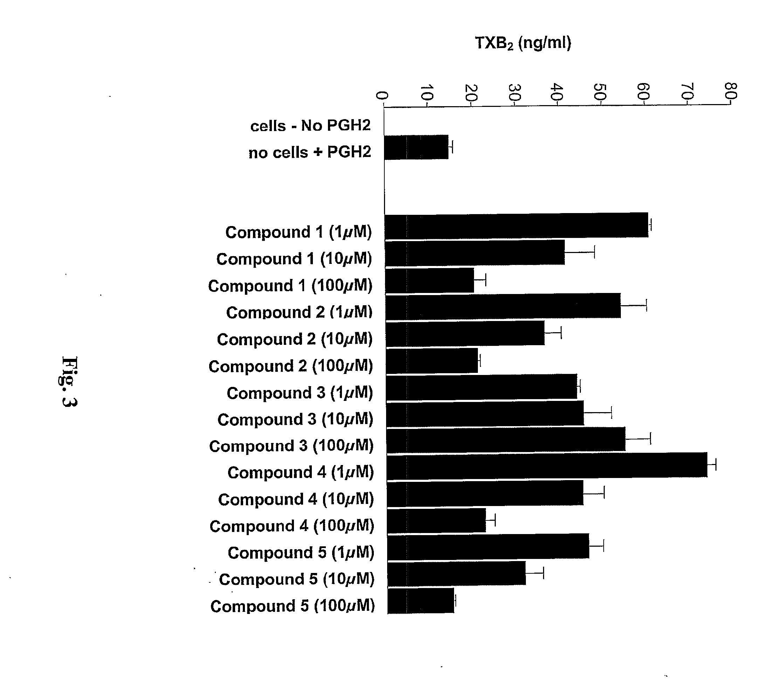

Test compounds at 0µM, 1µM, 10µM and 100µM (in DMSO) were incubated with undifferentiated U937 cells (6 x 10⁶/ml) for 30 min at 37°C, after which PGH₂ (5µM) was added. After incubation for 10 min, the reaction was terminated by centrifugation and the supernatants were collected and TXB₂ production was measured in triplicate by RIA. ANOVA followed by Newman-Keuls multiple comparisons test was used to examine differences between doses and the control values. A statistical significance level of 0.05 was used and differences from control values are indicated by an asterisk (*). The results are shown in Fig. 3.

Because none of these compounds inhibited PGE₂ in the initial assays it can be inferred that the inhibition demonstrated in these assays is specific to TXS. These results indicate that Compounds 1, 2, 4 and 5 are selective thromboxane synthase inhibitors, whereas the thromoboxane synthase activity displayed by Compound 3 was less specific.

### INHIBITION OF NFκB

Nuclear factor κB (NF-κB) is a nuclear transcription factor that regulates expression of a large number of genes (including COX-2) that are critical for the regulation of inflammation and various autoimmune diseases, as well as apoptosis, viral replication and tumorigenesis. NF-kB is activated by a variety of stimuli that include growth factors, cytokines, lymphokines, UV radiation, pharmacological agents and stress. Inhibition of NFκB by a test compound suggests that the compound will have anti-inflammatory activity *in vivo.*

Human macrophage THP-1 cells, modified to include the β-lactamase reporter gene, were seeded into wells of a 96-well plate (50 x 10³ cells/well) in the presence of RPMI 1640 medium (70µl). TNFα (7.5 ng/ml) and human sera (final concentration of 20%) were added to each well. Plates were incubated for 5h at 37°C to allow for NFκB-stimulated β-lactamase production. LiveBLAzerTM FRET B/G Substrate (CCF4-AM) was then added to assay for β-lactamase activity. Once CCFA-AM enters a cell, it is converted to negatively charged CCF4 by endogenous esterases. Excitation of this substrate at 409nm leads to efficient FRET between the coumarin and fluorescein moieties, resulting in a green fluorescence detectable at 530nm. The presence of β-lactamase leads to cleavage of CCF4 and results in a loss of FRET, resulting in a robust blue fluorescent signal detectable at 460 nm. Thus, activity of beta-lactamase (a marker of NFkB-promoter activity) is measured as a product to substrate ratio (blue/green fluorescence ratio: 460nm/530nm).

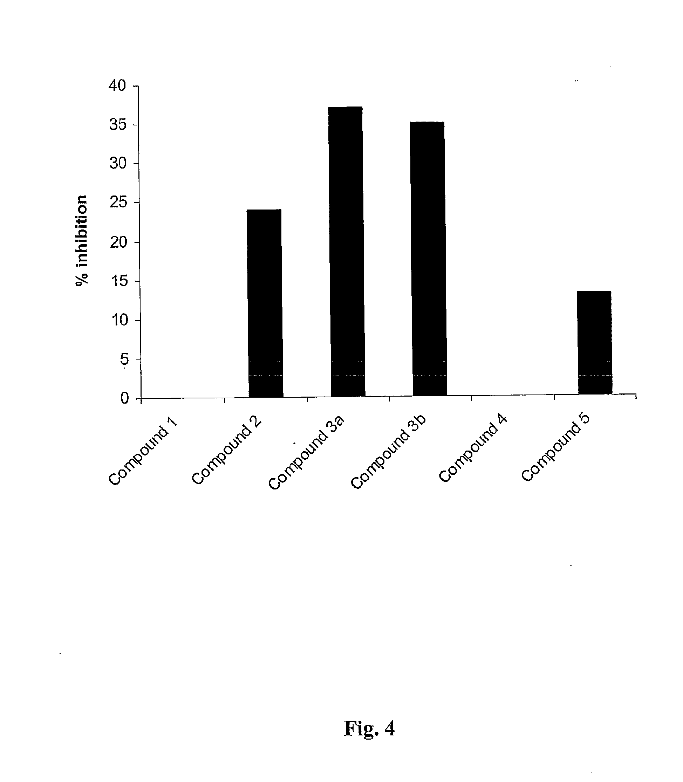

Compounds 2, 3 and 5 significantly (p < 0.001) inhibited NFκB when compared to vehicle control as shown in Fig. 4.

### INHIBITION OF NITRIC OXIDE

Nitric oxide (NO) plays an important regulatory/modulatory role in a variety of inflammatory conditions (Blantz and Munger 2002). Large amounts of NO are produced at sites of inflammation through the action of inducible nitric oxide synthase (iNOS) present in both infiltrating leucocytes and activated, resident tissue cells (Evans 1995). It can be vasodilatory and interfere with adhesion molecules to prevent neutrophil adhesion. NO release may also lead to the formation of highly reactive species such as peroxynitrite and stable nitrosothiols and may cause mitochondrial damage and nitration of protein tyrosine residues. Excessive NO is produced during the course of a variety of inflammatory and autoimmune diseases, including systemic lupus erythematosus (SLE), Sjögren's syndrome (SS), vasculitis, rheumatoid arthritis, and osteoarthritis (Clancy et al. 1998). Consequently, its inhibition is considered to be an anti-inflammatory strategy.

Exposure of various cells (e.g. macrophages and smooth muscle cells) to bacterial LPS and proinflammatory cytokines induces *i*NOS which generates NO. NO production can be indirectly quantified by measurement of nitrite (NO₂-), a stable breakdown product of NO. The mouse macrophage cell line RAW 264.7 was cultured in DMEM supplemented with FCS, 2mM glutamine and 50U/ml penicillin/streptomycin. Cells were treated with either test compound at 10µM (in 0.125% DMSO) or vehicle alone, together with 10ng/ml LPS. After incubation for 24hrs, culture media was collected and analysed immediately for NO concentration using the Griess reaction (Eigler et al. 1995).

Compounds 1, 3a and 3b inhibited NO synthesis at 10µM whereas Compounds 2 and 4 induced it. Compound 5 was not examined.

**Table 1. Effect of test compounds on NO synthesis**

| **Compound (10µM)** | **effect compared with vehicle control** | **difference between test group and vehicle** |
|---|---|---|
| Compound 1 | ↓by 9% | n.s. |
| Compound 2 | ↑by 5% | *p* = 0.023 |
| Compound 3a | ↓by 7% | n.s. |
| Compound 3b | ↓by 34% | *p* = 0.0001 |
| Compound 4 | ↑by 31% | *p* < 0.0001 |

These results indicate yet another mechanism by which the compounds are anti-inflammatory.

### ANTI-INFLAMMATORY ACTIVITY IN MURINE EAR INFLAMMATION

Compounds were examined for their ability to inhibit ear swelling in mice induced by the topical application of arachidonic acid (AA). The inflammatory response due AA, the immediate precursor of the eicosanoids, is due to formation of AA metabolites via both the cyclooxygenase (COX) and lipoxygenase (LOX) pathways (Young et al. 1984). AA induces an increase in both PGE₂ and LTC₄ synthesis which precedes the increase in ear thickness (Opas et al. 1985; Chang et al. 1986).

Female BALB/c mice (ARC, WA, Australia), weighing 15-21g were injected with Compound 2 delivered in polyethylene glycol (PEG) 400:phosphate buffered saline (PBS) 1:1 intraperitoneally (i/p) at a dose of 25mg/kg either 30min prior to or immediately before the application of AA to the ears. Mice were anaesthetised using isoflurane and baseline thickness of both ears was measured using a spring micrometer. Each mouse received a total of 20µL of AA in ethanol (50mg/ml) applied to the inner and outer surfaces of each pinna (i.e. 0.5mg AA per ear). Mice were anaesthetised again to remeasure the ears at 1hr post-AA application.

The difference in ear swelling pre- and post-application of AA for each ear was calculated, and the average for the two ears of each mouse calculated. The difference in mean swelling of each test group compared to the group given vehicle alone was calculated using a two-tailed unpaired *t* test (Prism 4, GraphPad Software).

All compounds resduced AA-induced ear inflammation. Treatment with Compounds 2, 3a and 3b resulted in a significant reduction in ear thickness compared with treatment with vehicle alone.

**Table 2. Change in ear thickness in response to the application of AA**

| **Compound** | **Increase in ear thickness (mean ± SD, x 0.01mm)** | **difference between test group and vehicle** |
|---|---|---|
| Vehicle | 14.5 ± 4.7 | - |
| Compound 1 | 9.5 ± 2.3 | *p* = 0.3221 |
| Compound 2 | 6.5 ± 2.9 | *p* = 0.0440 |
| Compound 3a | 6.7 ± 2.1 | *p* = 0.0362 |
| Compound 3b | 7.1 ± 3.0 | *p* = 0.0268 |
| Compound 4 | 9.2 ± 3.6 | *p* = 0.3381 |

These data demonstrate the anti-inflammatory activity of the test compounds *in vivo.*

### ANTI-INFLAMMATORY ACTIVITY IN MURINE MODEL OF THE UV IRRADIATION-INDUCED SKIN OEDEMA

Acute exposure of mammalian skin to UV irradiation causes an inflammatory reaction manifested by erythema and oedema. This reaction is mediated in part by pro-inflammatory prostaglandins (PGD₂, PGE₂, PGF_{2α} and possibly PGI₂) and leucotrienes, as well as the generation of reactive free radicals and reactive oxygen species (Sondergaard et al. 1985; Gonzalez and Pathak 1996; Widyarini et al. 2001).

Groups of 4-5 female Skh:hr-1 albino mice were irradiated with 1 x 3 MEdD (minimal edematous dose) of solar simulated UV radiation (SSUV). Solar-simulated ultraviolet radiation (SSUV) was provided by a planar bank of 6 UVA tube (Hitachi 40W F40T 10/BL, black light) and one UVB tube (Philips TL 40W/12RS) with radiation filtered through a sheet of 0.125 mm cellulose acetate (Eastman Chemical Products, Kingport, Tenn, USA) to give 2.96 x 10-4W/cm2 UVA and 1.59 x 10-5W/cm2 UVB. During irradiation, the cages were rotated below the lights to reduce the variation in radiation intensity in different positions.

Either test compound (0.2ml of a 20µM solution) or vehicle (propylene glycol /ethanol/water 1:2:1) was applied to the irradiated dorsal skin at 30 min, 2h and 4h post-irradiation. Dorsal skin fold measurements were made with a spring micrometer prior to and at 24hr and 48h post-UV exposure. The difference in skin thickness pre- and post-exposure to UVR was calculated for each mouse, and the differences examined between test compound and vehicle control were analysed using an unpaired two-tailed *t* test.

Skin fold thickening was evident at 24hrs post-UV irradiation and peaked at 48hrs, the last time point measured. Even though test compounds were applied only three times post-UV irradiation, and dosing was completed 20hrs prior to the first skin fold measurement, most of the compounds were active in reducing UV-induced inflammation, as highlighted in the tables and graphs below.

Topical administration of all compounds demonstrated a tendency to reduce UV-induced inflammation. Compound 3a significantly inhibited skin oedema at both time points. Compounds 1 and 2 significantly inhibited at 48hrs.

**Table 3. The change in dorsal skin fold thickness at 24hrs post-irradiation**

| **Compound** | **Increase in skin thickness (mean ± SD, x 0.01mm)** | **difference between test group and vehicle** |
|---|---|---|
| Vehicle | 78 ± 23 | - |
| Compound 1 | 54 ± 20 | *p* = 0.0579 |
| Compound 2 | 68 ± 24 | *p* = 0.4275 |
| Compound 3a | 32 ± 18 | *p* = 0.0023 |
| Compound 3b | 56 ± 14 | *p* = 0.0671 |
| Compound 4 | 63 ± 22 | *p* = 0.2407 |

**Table 4. The change in dorsal skin fold thickness at 48hrs post-irradiation**

| **Compound** | **Increase in skin thickness (mean ± SD, x 0.01mm)** | **difference between test group and vehicle** |
|---|---|---|
| Vehicle | 147 ± 37 | - |
| Compound 1 | 91 ± 7.6 | *p* = 0.0041 |
| Compound 2 | 105 ± 22 | *p* = 0.0299 |
| Compound 3a | 69 ± 24 | *p* = 0.0011 |
| Compound 3b | 113 ± 25 | *p* = 0.0746 |
| Compound 4 | 145 ± 11 | *p* = 0.9055 |

These results clearly demonstrate the anti-inflammatory activity of Compounds 1, 2 and 3a. Even though they were administered topically and within a short interval after the induction of inflammation, their effects were still evident up to 48 hrs later.

### ANTI-INFLAMMATORY ACTIVITY IN THE RAT AIR POUCH ASSAY

An alternative assay used to measure anti-inflammatory efficacy is the air pouch model which involves the repeated subcutaneous injection of air into the dorsum of rats followed 24h later by the intrapouch injection of an inflammatory stimulus (Gilroy et al. 1998).

Air pouches were raised on the dorsum of female Dark Agouti rats, approximately 7 weeks of age. To promote the formation of a cellular membrane lining the inside of each pouch, the pouches were maintained by re-inflating on days 2 and 5 after the initial injection of air. On re-inflation, the pouch was first deflated to ensure the needle was positioned correctly, before being re-inflated with 2 mL of sterile air. Using this protocol, the pouches remained inflated until use on day 7, when they were injected with 0.5 ml of either test compound at 100µM or vehicle control. After 15min, air pouches were injected with serum-treated zymozan (STZ - 500µg). Lavage of the air pouch (4 x 2ml lavages) was performed at 4h and leucocytes counted, after which the rats were killed, the air pouch excised and processed in formalin for histology. The sections were blinded to the person counting. Using a graticule with 100 squares and the 40x objective, the number of polymorphs were counted in the pouch lining at 10 different and non-adjacent sites. Group sizes were 5-6 rats. Data were analysed for statistical significance within each experiment and using an unpaired *t* test.

In general, there was concordance between the two separate measures of the extent of the inflammatory infiltrate (the numbers of leucocytes in lavage fluid and polymorphs in tissue sections). There was also concordance between the two measures for the effects of the test compounds, which strengthens the validity of each data set.

Most compounds tested demonstrated anti-inflammatory activity. At 1mM application, all compounds except Compounds 1 and 3a significantly suppressed the number of leucocytes in the lavage fluid. Compounds 2, 3a and 4 suppressed the number of polymorphs in the tissue sections.

**Table 5. Effect of instilling test compounds into inflammed rat air pouches**

| | **Leucocytes in lavage fluid (x 10⁻⁷)** | **Polymorphs in tissue sections (per 100 squares)** |
|---|---|---|
| **Compound 1** Vehicle | 0.89 ± 0.42. | 31.0 ± 7.25 |
| | 0.87 ± 0.28 | 31.1 ± 10.9 |
| | *p* = 0.9181 | *p* = 0.9921 |
| **Compound 2** Vehicle^{a} | 0.68 ± 0.36 | 14.3 ± 5.7 |
| | 1.41 ± 0.25 | 28.9 ± 6.6 |
| | *p* = 0.002^{b} | *p* = 0.002 |
| **Compound 3a** Vehicle | 0.47 ± 0.4 | 17.9 ± 14.2 |
| | 1.72 ± 1.3 | 27.0 ± 17.9 |
| | *p* = 0.1124 | *p* = 0.2196 |
| **Compound 3b** Vehicle | 0.66 ± 0.23 | ND |
| | 1.22 ± 0.41 | |
| | *p* = 0.0156 | |
| **Compound 4** Vehicle | 1.00 ± 0.38 | 23.4 ± 8.8 |
| | 2.23 ± 1.08 | 34.9 ± 14.6 |
| | *p* = 0.025 | *p* = 0.13; *p* = 0.065 (1-tailed) |

| | | |
|---|---|---|
| "Control was DMSO / PBS, the vehicle for the test compounds. The ratio of DMSO/PBS was 1:100. ^{b}unpaired t-test; 2-tailed unless otherwise indicated | | |

These results clearly demonstrate the anti-inflammatory activity of Compounds 2, 3a, 3b and *4 in vivo.*

### ANTI-INFLAMMATORY ACTIVITY IN THE MURINE MODEL OF DSS-INDUCED COLITIS

### Methods

Female BALB/c mice, 6-7 weeks old, were housed for a minimum of one week before any experimental work began. To induce colitis, dextran sulphate sodium (DSS - molecular weight 40kD, TdB Consultancy AB, Uppsala, Sweden (Dieleman et al. 1994), was administered fresh daily in the drinking water at a concentration of 4.5 - 5% for 5 days (Okayasu et al. 1990), after which water alone was given. This regime induced mild colitis, evidenced by diarrhoea and rectal bleeding, the severity of which was scored, as well as a reduction in the length of the colon. Histologically, colonic inflammation was evidenced by ulceration and an infiltration of neutrophils into the mucosa, which was also scored. The systemic effect of the colitis causes weight loss, which was also monitored.

### Results

In a series of experiments, oral administration of either Compound 1, Compound 2, Compound 3a, Compound 3b or Compound 4 reduced colonic inflammation. Importantly, the dose rate at which these compounds were active was relatively low - 1.0 -1.25mg/kg, given as a single oral dose per day.

### Experiment 1

Either vehicle (PEG 400:PBS 1:1) Compounds 1 or 4 were administered at a dose rate of 1mg/kg throughout the experiment until the mice were killed at day 17. Both compounds decreased colitis. In this assay, Compound 1 offered the better anti-inflammatory activity (based on a reduction in colonic ulceration, a delay in the onset of clinical signs, a reduction in the clinical score, a reduction in the occurrence and severity of clinical signs, reduced body weight loss and reduced time to recovery) than Compound 2.

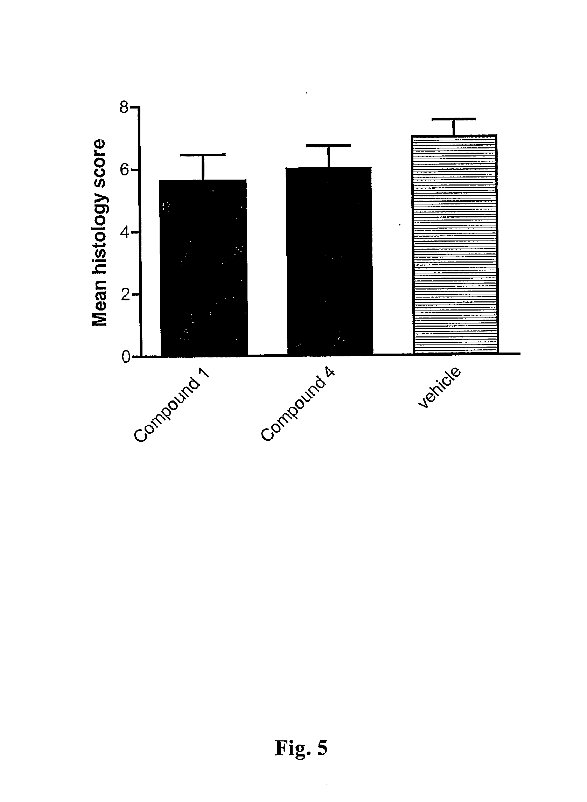

The mean histology scores are shown Fig. 5, suggesting less colitis in those mice treated with Compounds 1 and 4. There was statistically significant (p = 0.0135) less ulceration of the colonic epithelium from those mice given Compound 1 compared with those given vehicle alone.

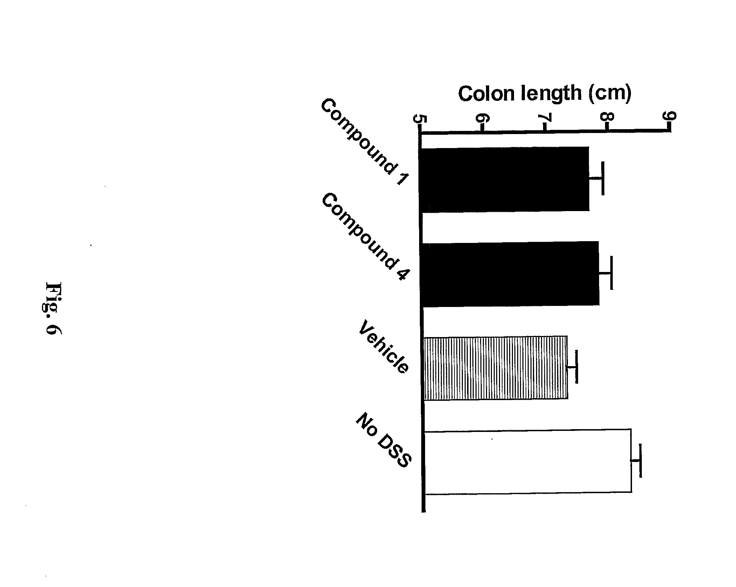

The colonic inflammation induced by ingestion of DSS causes shortening of the colon, and thus protection conferred by test agents might be evidenced by a reduction in this shortening. There was a trend towards the colons from the mice given Compound 1 (p = 0.1922) or Compound 4 (p = 0.0542) to be longer than those from the mice given vehicle alone. See Fig. 6.

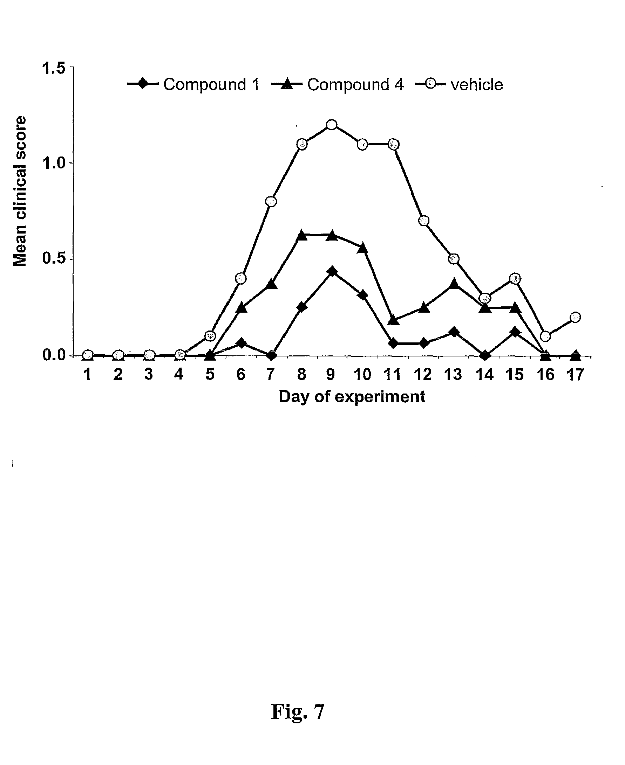

The clinical signs appeared in several mice in the groups given vehicle at day 5, but were not evident until day 6 in the groups given either Compounds 1 and 4, suggesting that those compounds may have delayed the onset of colitis. By day 11, the mean clinical score for the groups given either Compounds 1 and 4 was significantly lower (*p* = 0.017 and *p* = 0.043 respectively) than that of the group given vehicle alone. These results suggest that both Compounds 1 and 4 were anti-inflammatory. See Fig. 7.

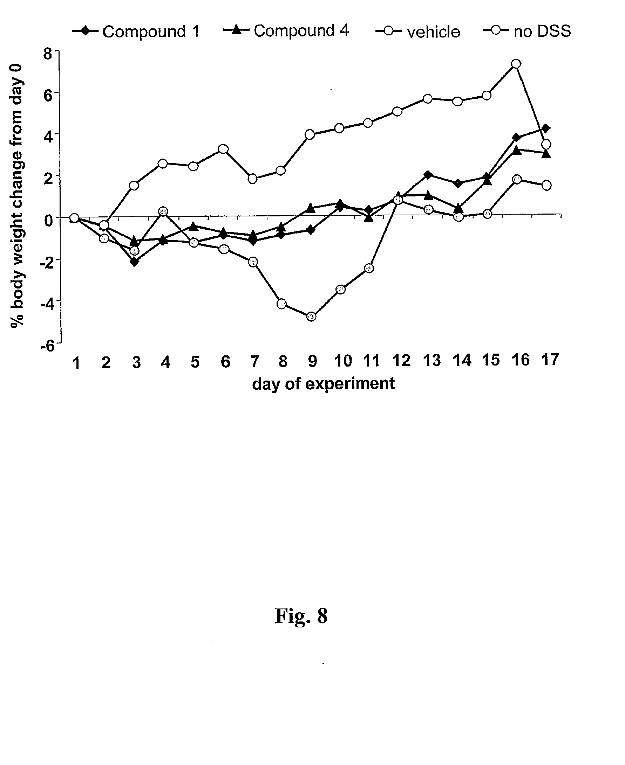

The maximum mean weight loss was only 1% for Compound 4 and 2% for Compound 1 but 5% for the group given vehicle alone. The switch from mean body weight loss to mean body weight gain for a particular group suggests in part, that resolution of colitis for that group has occurred. This occurred at day 10 for the group given Compound 1, at day 12 for Compound 4, but not until day 15 for the vehicle. See Fig. 8.

### Experiment 2

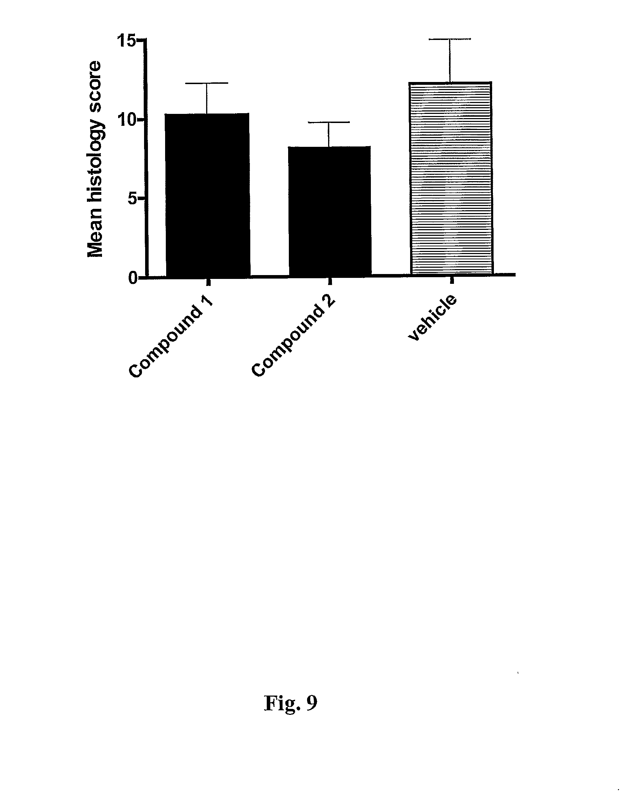

Either Compounds 1 and 2, at a dose of 1mg/kg or vehicle were administered for 10 days prior to DSS being added to the drinking water to induce colitis, after which test compound was stopped. DSS was administered for 5 days and the mice killed 8 days later (day 23). Inflammation was reduced by both compounds, even though they were administered only prior to but not during its induction. See Fig. 9.

The amount and severity of mucosal ulceration was greater in the vehicle-treated group, but not significantly so.

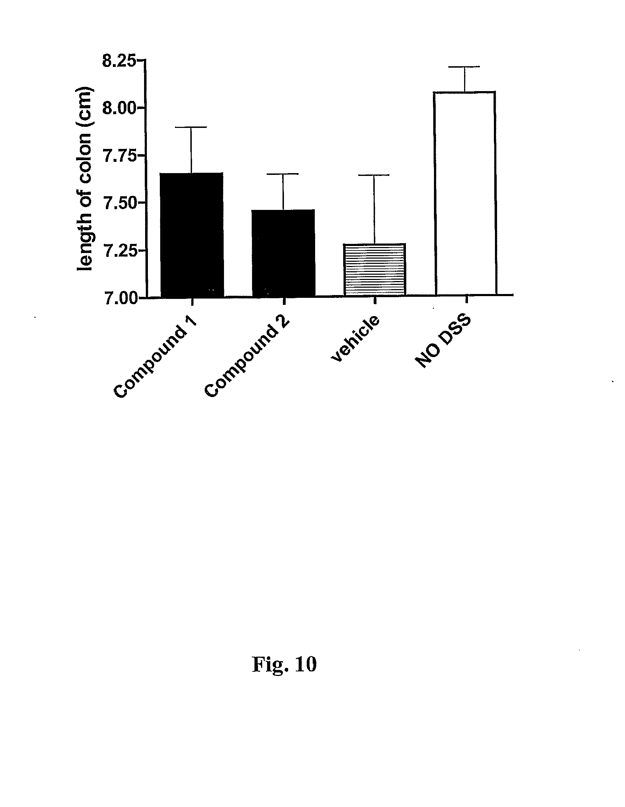

Whilst there was a trend, there were no significant differences in colon length between either of the test groups and vehicle. There was a significant difference between the colon lengths of the mice which were not given DSS and vehicle group (p = 0.0414). However, the colons of the Compound 1-treated groups were not statistically different in length from those of the control group, suggesting that Compound 1 protected against the inflammatory effects of DSS ingestion to some extent. See Fig. 10.

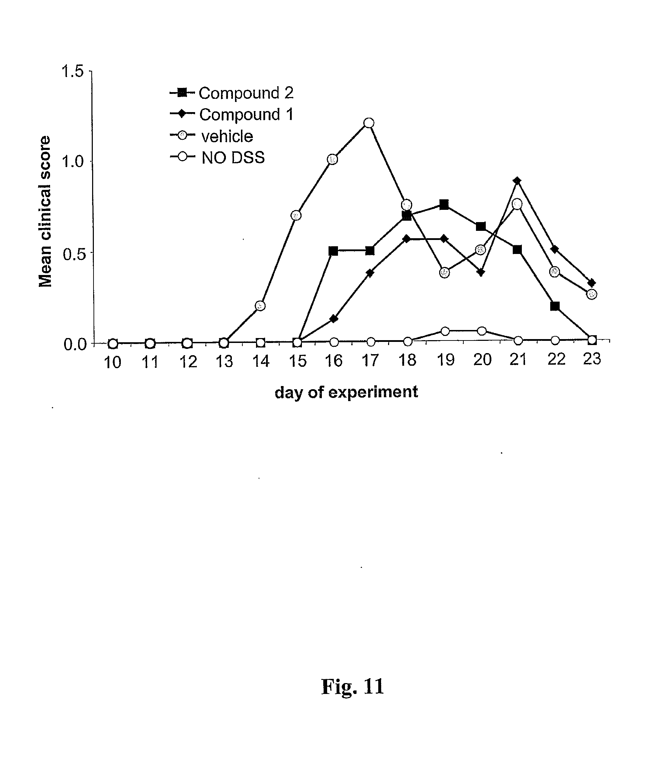

One mouse in the group treated with vehicle died at day 18 whereas no mice died in either of the treatment groups. Although not statistically significant when compared with the vehicle-treated group, there was solid evidence of a reduction in the clinical signs of colitis in both treatment groups. See Fig. 11.

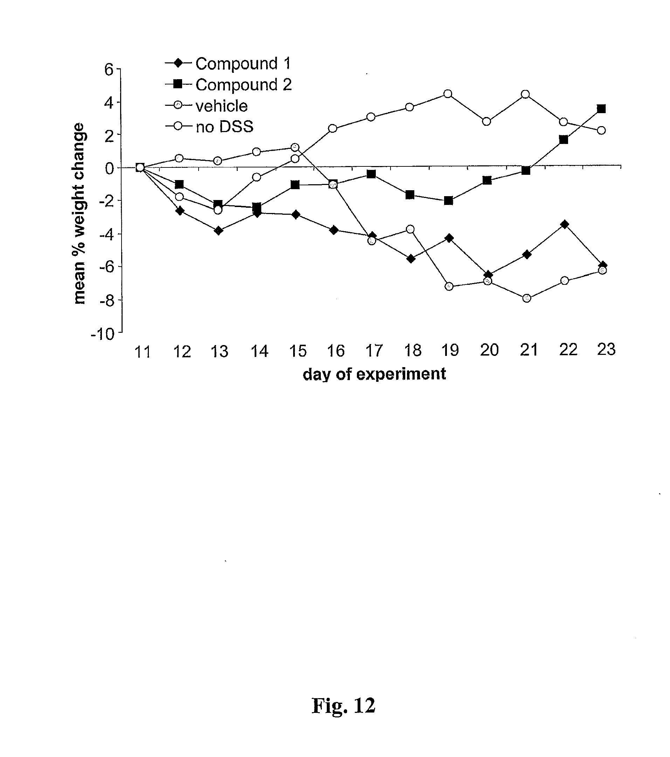

Administration of Compound 2, and to a lesser extent Compound 1 reduced the body weight loss associated with DSS-induced colonic inflammation. By day 22, the mice receiving Compound 2 weighed significantly more than those receiving vehicle (p = 0.0291). See Fig. 12.

### Experiment 3

Either the *cis* or the *trans* isomers of Compound 3 (Compound 3a and 3b respectively) at 1.25mg/kg or vehicle were administered throughout the experiment until the mice were killed at day 17. Based on the clinical indicators, colonic length and histological changes, dosing with Compound 3a and to a lesser extent Compound 3b reduced colonic inflammation and hastened its resolution.

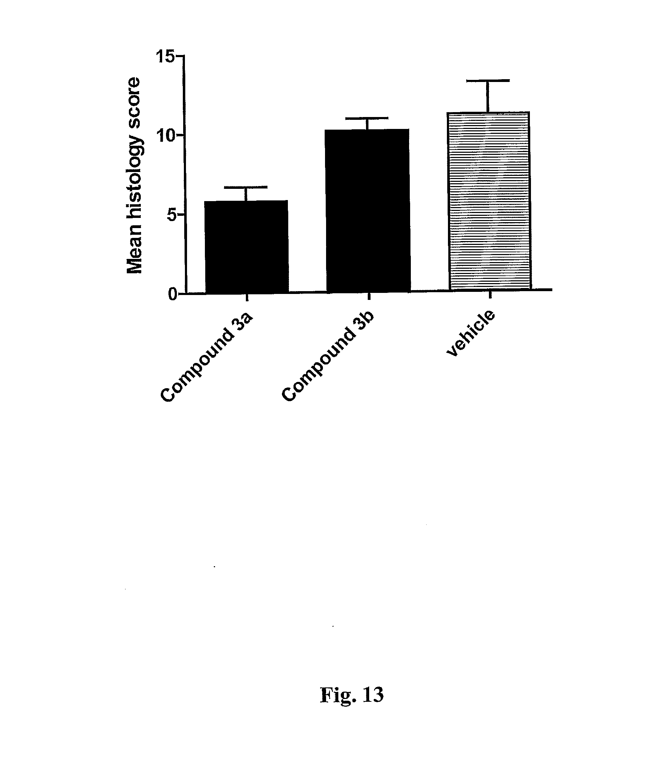

Ulceration of the posterior colon was not observed in the group given Compound 3a and only in 1/8 mice given Compound 3b. However, ulceration was observed in 3/8 mice given vehicle alone. The degree of mucosal damage for those mice given Compound 3a was significantly lower (*p* = 0.018) than that experienced by the vehicle-treated group. See Fig. 13.

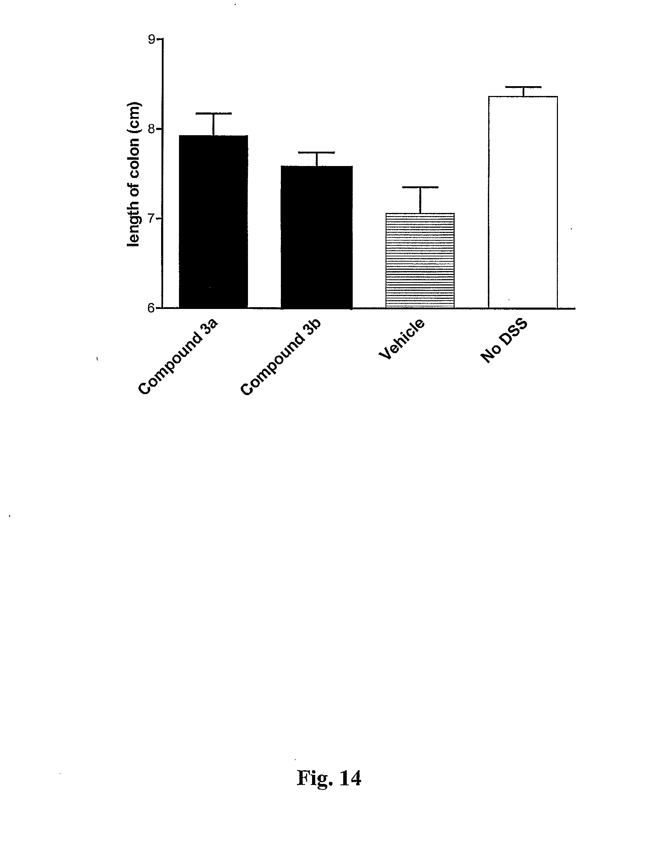

The colons from the group given Compound 3a were significantly longer (*p* = 0.039) than those from the group given vehicle alone. See Fig. 14.

The mean score of the group receiving Compound 3a was significantly lower than that of the vehicle group throughout days 8 (*p* = 0.0078), 9 (*p* = 0.0326), 10 (*p* = 0.0386), and 11 (*p* = 0.0252). However, as the colitis began to resolve in the vehicle-treated mice, that difference was less evident, and lost statistical significance. However, by the final days of the experiment, the mean clinical score was again significantly lower for the group receiving Compound 3a on both days 15 (*p* = 0.0033) and 16 (*p* = 0.0016). This suggested Compound 3a exerted an anti-inflammatory effect and hastened the resolution of inflammation.

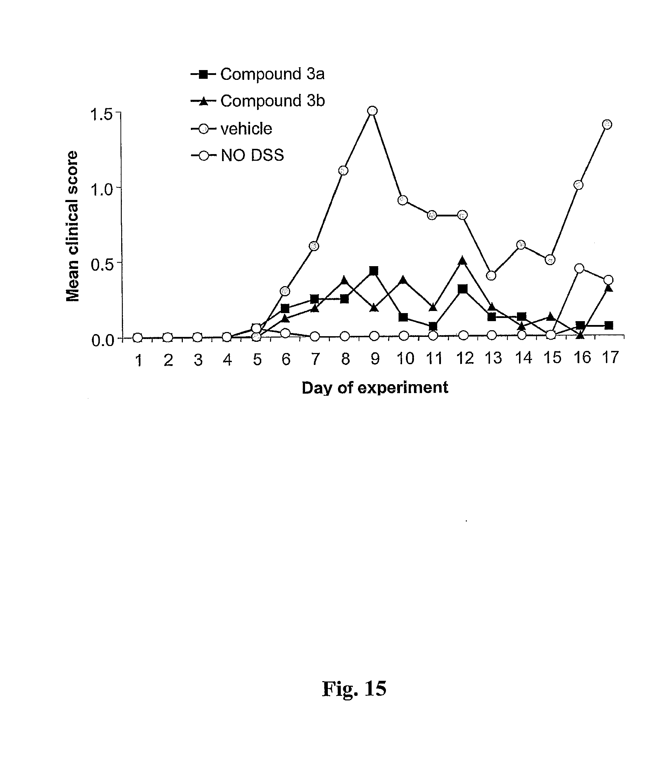

Similarly, the mean score of the group receiving Compound 3b was significantly lower than that of the vehicle group throughout days 8 (*p* = 0.0055), 9 (*p* = 0.0057) and 11 (*p* = 0.0252). The mean clinical score was again significantly lower for the group receiving Compound 3b on 17 (*p* = 0.0177). This suggested that oral dosing with Compound 3b also had an anti-inflammatory effect and hastened the resolution of inflammation. See Fig. 15.

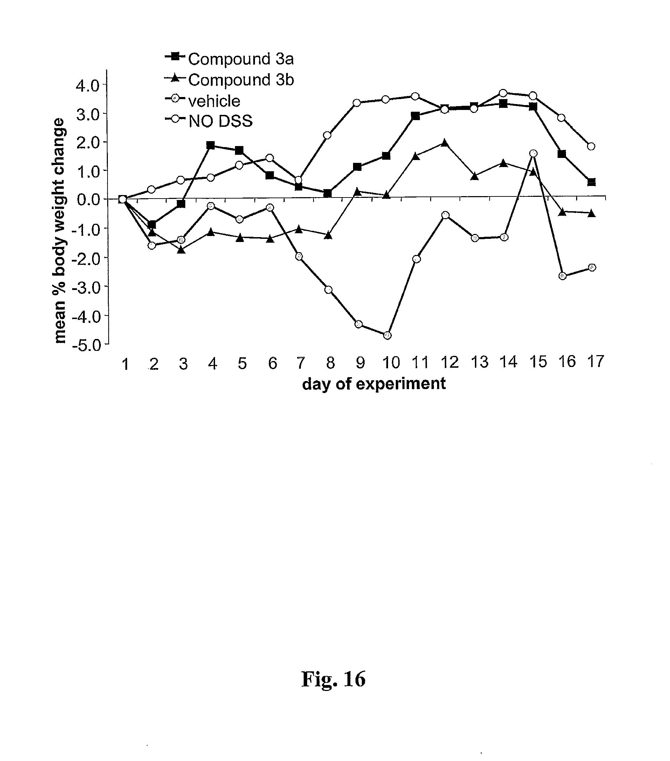

Whilst the trends were clear, there were no statistically significant differences in body weight change between the groups either isomer of Compound 3 and the group given vehicle. The maximum mean weight loss was less than 2% for the groups given Compound 3a, whereas it was nearly 5% for the group given vehicle alone. The switch from mean body weight loss to mean body weight gain occurred relatively quickly at day 4 for Compound 3a but not in a substantial way at all for the vehicle group. See Fig. 16.

### Experiment 4

Groups of mice (*n* = 5) were dosed once daily with Compound 1 1mg/kg or vehicle throughout the experiment, and killed either on day 6, 9 or 13. Colons were removed, opened longitudinally and washed in PBS with pen/strep (100U/ml/100µg/ml, Invitrogen) with added fungizone (2.5ug/ml, Invitrogen). They were then cut into small portions and approximately 100mg colon was placed into one well of a 24-well plate either in the presence or absence of ConA (10µg/ml, Sigma) containing 1ml modified RPMIc (pen/strep and fungizone as above) for 18-24 hours. Supernatant was collected, centrifuged and stored at -80°C. PGE₂ and TXB₂ in the culture supernatants were **measured in triplicate by ELISA (Cayman Chemical).**

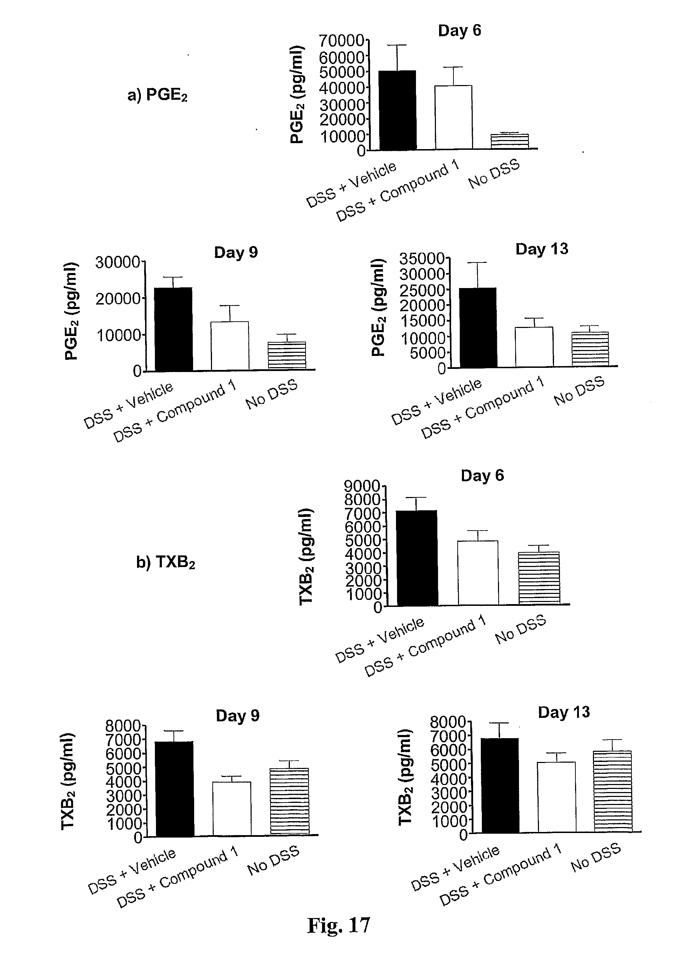

By culturing the colons in the absence of mitogenic stimulation, an assessment of the inflammatory mediators being produced currently can be made. The inflamed colons from mice exposed to DSS tend to produce more inflammatory mediators than healthy, uninflamed colons (Dieleman et al. 1998; Tomoyose et al. 1998; Morteau et al. 2000; Micallef et al. 2002). In this experiment, where DSS had been ingested, the colons produced significantly more PGE₂ at day 9 (*p* < 0.05) and more TXB₂ at day 6 (*p* < 0.05), when compared with the colons from healthy mice without colitis. There were, however, trends towards the colons of mice given DSS to produce more PGE₂ and TXB₂ throughout the entire experiment, particularly in the early phase. The effect of Compound 1 at ameliorating these inflammatory responses was assessed by comparing results from mice given DSS and treated with Compound 1 with those given DSS and treated with vehicle alone. The colons from the Compound 1-treated mice tended to produced less PGE₂ at day 9 and day 13, and less TXB₂ at day 6 and day 9 (*p* < 0.05) - see figure 17.

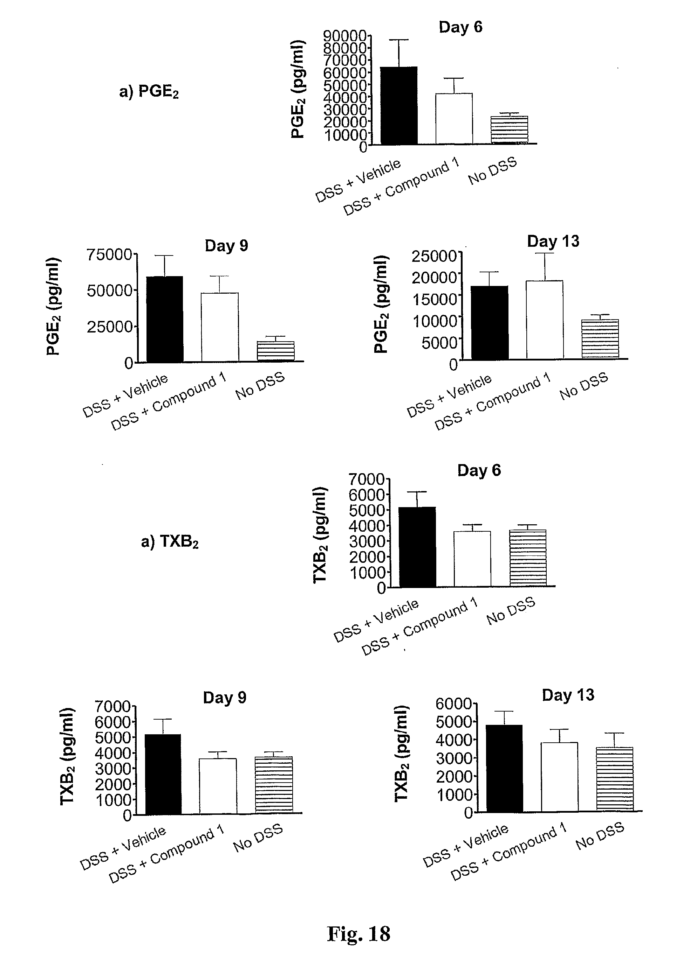

When colons were cultured in the presence of a mitogen, their ability to respond to inflammatory/mitogenic stimuli was being evaluated. Where DSS had been ingested, the colons produced significantly more PGE₂ at day 9 (*p* < 0.05) at day 6 in response to ConA, when compared with the colons from healthy mice without colitis. Again, there were trends towards the stimulated colons from mice given DSS to produce more PGE₂ and TXB₂ throughout the entire experiment, particularly in the early phase. By day 13, there were no differences between healthy and inflamed colons, suggesting that the acute inflammation caused by DSS had begun to resolve. There was a trend for treatment with Compound 1 to reduce these responses, although not significantly so. See figure 18.

These results demonstrate that the anti-inflammatory effect of oral dosing with Compound 1 in the colitis modelwas probably due to its effect at attenuating eicosanoid synthesis by the inflamed colon.

### CARDIOPROTECTIVE ACTIVITY VASODILATORY ACTIVITY IN THE RAT AORTIC RING ASSAY

The vasodilatory capacity of test compounds was examined *ex vivo* using the rat aortic ring assay. The addition of noradrenaline to the test bath causes the rings to contract, and if that vasoconstriction is inhibited by a test agent ie it antagonises the effect of noradrenaline, it suggests that that agent may have vasodilatory anti-hypertensive activity.

### Methods

Male Sprague-Dawley rats (250 ± 50g) were euthanased with 80% CO₂ and 20% O₂. The thoracic aorta was excised and quickly mounted in organ-baths as described (Chin-Dusting et al. 2001). Full concentration-contractile curves were obtained to noradrenaline (0.1nM-10mM) with and without test compounds delivered at a concentration of 1µg/ml. Experiments were repeated in *n* = 5 different rings from 5 different animals. Only one compound at any one concentration was tested on any one ring from any one animal. Sigmoidal dose response curves were fitted for the data and the logEC₅₀ calculated (Prism 4, GraphPad Software). The difference in these values between the presence and absence of test compound was calculated using a two-tailed paired *t* test. The effects of β-oestrodiol and vehicle alone were examined as a positive and negative control respectively.

### Results

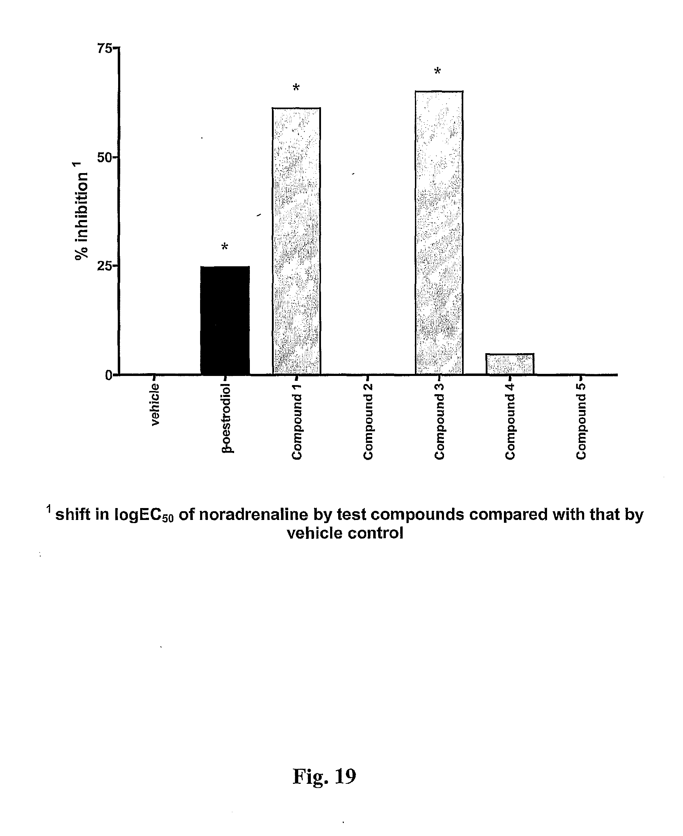

Compounds 1, 2,4, 5 and the isomeric mixture of Compound 3 were examined in this assay. Compounds 1 and 3 significantly inhibited the contractile response (logEC₅₀) of the aortic ring to noradrenaline compared with vehicle alone. These data indicate that these compounds, in addition to possessing anti-inflammatory activity, may have cardiovascular activity as well. See Fig. 19.

### ABSENCE OF GASTROINTESTINAL TOXICITY

### Compound 1

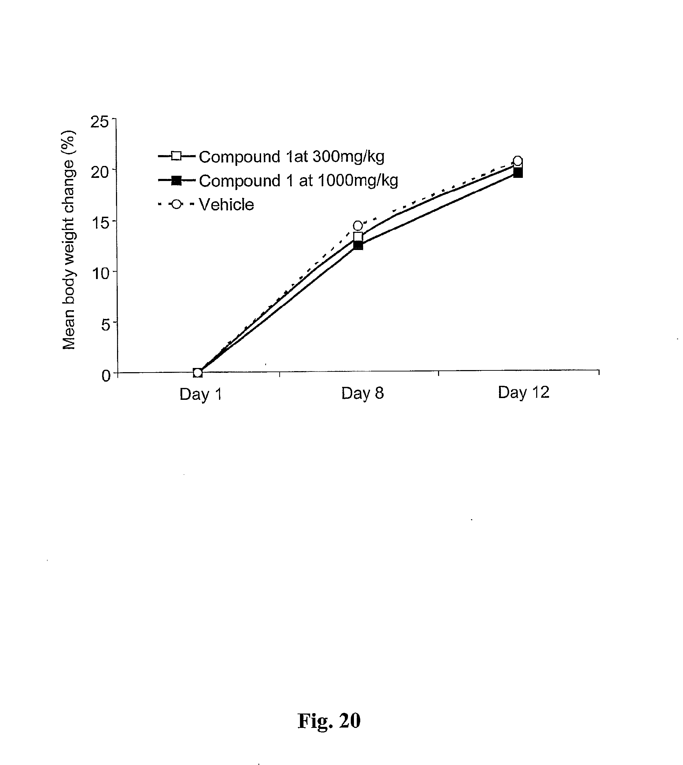

Oral toxicity of Compound 1 when administered daily for 11 days was investigated. Compound 1 in CMC 0.1% as administered at the very high doses of 300mg^{c}/kg and the limit dose of 1000mg/kg to groups of four Sprague Dawley rats (two of each gender). Vehicle was administered to a third group. See Fig. 20.

The formulation of Compound 1 in CMC 0.1 % was stable over a seven day period at both 4°C, 25°C and 40°C. Fresh batches of Compound 1 were supplied after day seven. Bodyweights were measured immediately before administration, then at day eight and again at culling on day 12. All rats were observed daily for signs of toxicity throughout the experimental period. At necropsy, gross examination of the external surface of the body, all orifices and the thoracic and abdominal cavities and their contents was carried out. Liver, kidney, adrenals, spleen and gonads were weighed. Terminal haematology, serum biochemistry and histology of the liver, spleen and bone marrow was done.

There was no evidence of toxicity of any kind, in particular gastrointestinal disturbance, weight loss, coagulopathy, anaemia or other blood dyscrasia which would suggest gastrointestinal bleeding.

### Compound 2

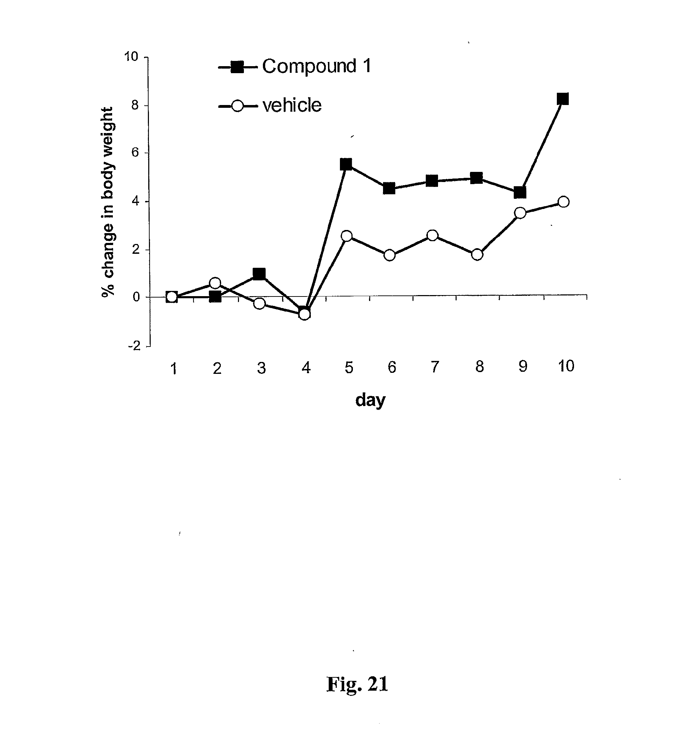

Compound 2 at 5mg/kg (*n* = 3) or vehicle (PEG 400:PBS 1:1; *n* = 2) was administered via gavage to female BALB/c mice for 10 days. There were no adverse clinical signs in either group, and body weight changes were similar. There were no gross pathological changes evident at necropsy. Histology of caecum and colon were normal. See Fig. 21.

### Compound 3

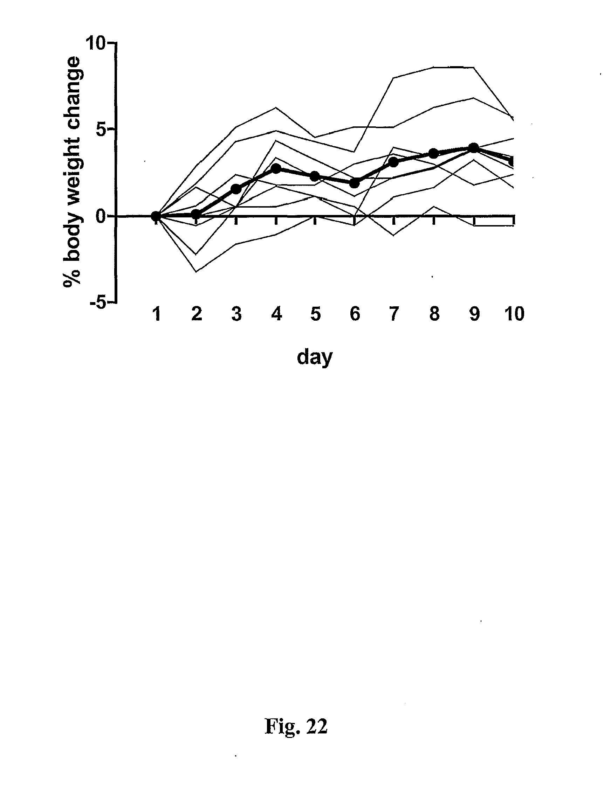

The diastereoisomeric mixture of Compounds 3a and 3b in PEG 400:PBS 1:1 was administered to 7 week old female BALB/c mice (*n* = 8) by gavage at 20mg/kg daily for 10 days. There was no vehicle control group for comparison. However, all mice maintained body weight and there was a mean increase in the group over the 10 days of 5% of the starting weight, which was typical of mice of that age. See Fig. 22.

### Compound 4

Compound 4 at 5mg/kg (*n* = 3) or vehicle (*n* = 2, PEG:PBS 1:1) was administered via gavage to female BALB/c mice for 10 days. There were no adverse clinical signs in either group, and body weight changes were similar. There were no gross pathological changes evident at necropsy. Histology of caecum and colon were normal.

These data confirm that the compounds do not cause gastrointestinal toxicity as evidenced by normal weight gain, absence of clinical signs and absence of histopathological changes in those animals receiving them over prolonged periods and often at extremely high doses.

To the extent that the word comprising in used in this specification it is to be understood that the invention in the alternative also extends to a combination consisting of or consisting essentially of said elements or integers.

The reference to any prior art in this specification is not, and should not be taken as, an acknowledgment or any form of suggestion that that prior art forms part of the common general knowledge in the field of endeavour.

Those skilled in the art will appreciate that the invention described herein is susceptible to variations and modifications other than those specifically described. It is to be understood that the invention includes all such variations and modifications. The invention also includes all of the steps, features, compositions and compounds referred to or indicated in this specification individually or collectively, and any and all combinations of any two or more of said steps or features.

### References

Carty, E., et al. (2000). "Ridogrel, a dual thromboxane synthase inhibitor and receptor antagonist: anti-inflammatory profile in inflammatory bowel disease." Alimentary Pharmacology & Therapeutics 14(6): 807-17.
Carty, E., et al. (2002). "Thromboxane synthase immunohistochemistry in inflammatory bowel disease." Journal of Clinical Pathology 55(5): 367-370.
Caughey, G. E., et al. (2001). "Roles of cyclooxygenase (COX)-1 and COX-2 in prostanoid production by human endothelial cells: selective up-regulation of prostacyclin synthesis by COX-2." Journal of Immunology 167(5): 2831-8.
Cohn, S. M., et al. (1997). "Crypt stem cell survival in the mouse intestinal epithelium is regulated by prostaglandins synthesized through cyclooxygenase-1." Journal of Clinical Investigation. 99(6): 1367-79.
Dogne, J. M., et al. (2004). "New developments on thromboxane and prostacyclin modulators part I: thromboxane modulators." Current Medicinal Chemistry 11(10): 1223-41.
Gilroy, D. W., et al. (1999). "Inducible cyclooxygenase may have anti-inflammatory properties.[comment]." Nature Medicine. 5(6): 698-701.
Hawkey, C. J., et al. (1985). "Modulation of human colonic arachidonic acid metabolism by sulfasalazine." Digestive Diseases & Sciences 30(12): 1161-5.
Levi, S., et al. (1990). "Inhibitory effect of non-steroidal anti-inflammatory drugs on mucosal cell proliferation associated with gastric ulcer healing." Lancet 336(8722): 1073.
Lianos, E. A. and B. A. Bresnahan (1999). "Effect of thromboxane A2 inhibition and antagonism on prostaglandin and leukotriene synthesis in glomerular immune injury." Journal of Laboratory & Clinical Medicine 134(5): 478-82.
McCartney, S. A., et al. (1999). "Selective COX-2 inhibitors and human inflammatory bowel disease." Alimentary Pharmacology & Therapeutics 13(8): 1115-7.
Miller, T. A. (1983). "Protective effects of prostaglandins against gastric mucosal damage: current knowledge and proposed mechanisms." Am J Physiol Gastrointest Liver Physiol 245: G601-G623.
Penglis, P. S., et al. (2000). "Differential regulation of prostaglandin E2 and thromboxane A2 production in human monocytes: implications for the use of cyclooxygenase inhibitors." Journal of Immunology 165(3): 1605-11.
Rampton, D. S. and C. E. Collins (1993). "Review article: thromboxanes in inflammatory bowel disease--pathogenic and therapeutic implications." Alimentary Pharmacology & Therapeutics 7(4): 357-67.
Raud, J., et al. (1988). "Enhancement of acute allergic inflammation by indomethacin is reversed by prostaglandin E2: apparent correlation with in vivo modulation of mediator release." Proc. Natl. Acad. Sci. USA. 85: 2315-2319.
Robert, A., et al. (1979). "Cytoprotection by prostaglandins in rats. Prevention of gastric necrosis produced by alcohol, HCl, NaOH, hypertonic NaCl, and thermal injury." Gastroenterology 77(3): 433-43.
Sakamoto, C., et al. (1993). "PGE2 protects isolated cells against injury through multiple mechanisms." Gastroenterologia Japonica 28 Suppl 5: 122-6.
Shi, H., et al. (1998). "Effect of thromboxane A2 inhibitors on allergic pulmonary inflammation in mice." European Respiratory Journal 11 (3): 624-9.
Takeuchi, K., et al. (2001). "Adaptive gastric cytoprotection is mediated by prostaglandin EP1 receptors: a study using rats and knockout mice." Pharmacology and Experimental Therapeutics 297(3): 1160-1165.
Tessner, T. G., et al. (2004). "Prostaglandin E2 reduces radiation-induced epithelial apoptosis through a mechanism involving AKT activation and bax translocation." J. Clin. Invest 114: 1676-1685.
Thomas, D. W., et al. (2003). "Proinflammatory actions of thromboxane receptors to enhance cellular immune responses." Journal of Immunology 171(12): 6389-95.
Tilley, S. L., et al. (2001). "Mixed messages: modulation of inflammation and immune responses by prostaglandins and thromboxanes." Journal of Clinical Investigation 108(1): 15-23.
Vane, J. R. and R. M. Botting (2003). "The mechanism of action of aspirin." Thrombosis Research 110(5-6): 255-8.
Whittle, B. J. and J. Lopez-Belmonte (1993). "Actions and interactions of endothelins, prostacyclin and nitric oxide in the gastric mucosa." Journal of Physiology & Pharmacology 44(2): 91-107.
Blantz, R. C. and K. Munger (2002). "Role of nitric oxide in inflammatory conditions." Nephron. 90(4): 373-8.
Chang, J., et al. (1986). "Correlation between mouse skin inflammation induced by arachidonic acid and eicosanoid synthesis." Inflammation 10(3): 205-14.
Clancy, R. M., et al. (1998). "The role of nitric oxide in inflammation and immunity." Arthritis & Rheumatism. 41(7): 1141-51.
Demasi, M., et al. (2000). "Assay of cyclooxygenase-1 and 2 in human monocytes." Inflammation Research 49(12): 737-43.
Dieleman, L. A., et al. (1998). "Chronic experimental colitis induced by dextran sulphate sodium (DSS) is characterized by Th1 and Th2 cytokines." Clinical & Experimental Immunology 114(3): 385-91.
Dieleman, L. A., et al. (1994). "Dextran sulfate sodium-induced colitis occurs in severe combined immunodeficient mice." Gastroenterology 107(6): 1643-52.
Eigler, A., et al. (1995). "Exogenous and endogenous nitric oxide attenuates tumor necrosis factor synthesis in the murine macrophage cell line RAW 264.7." Journal of Immunology 154(8): 4048-54.
Evans, C. H. (1995). "Nitric oxide: what role does it play in inflammation and tissue destruction?" Agents & Actions - Supplements. 47: 107-16.
Gilroy, D. W., et al. (1998). "Differential effects of inhibition of isoforms of cyclooxygenase (COX-1, COX-2) in chronic inflammation.[comment]." Inflammation Research. 47(2): 79-85. Gonzalez, S. and M. A. Pathak (1996). "Inhibition of ultraviolet-induced formation of reactive oxygen species, lipid peroxidation, erythema and skin photosensitization by polypodium leucotomos." Photodermatol Photoimmunol Photomed 12(2): 45-56.
Micallef, M. J., et al. (2002). "The natural plant product tryptanthrin ameliorates dextran sodium sulfate-induced colitis in mice." International Immunopharmacology. 2(4): 565-78.
Morteau, O., et al. (2000). "Impaired mucosal defense to acute colonic injury in mice lacking cyclooxygenase-1 or cyclooxygenase-2." Journal of Clinical Investigation 105(4): 469-78.
Okayasu, I., et al. (1990). "A novel method in the induction of reliable experimental acute and chronic ulcerative colitis in mice." Gastroenterology 98(3): 694-702.
Opas, E. E., et al. (1985). "Prostaglandin and leukotriene synthesis in mouse ears inflamed by arachidonic acid." Journal of Investigative Dermatology 84(4): 253-6.
Sondergaard, J., et al. (1985). "Eicosanoids in skin UV inflammation." Photo-Dermatology 2(6): 359-66.
Tomoyose, M., et al. (1998). "Role of interleukin-10 in a murine model of dextran sulfate sodium-induced colitis." Scandinavian Journal of Gastroenterology 33(4): 435-40.
Widyarini, S., et al. (2001). "Isoflavonoid compounds from red clover (Trifolium pratense) protect from inflammation and immune suppression induced by UV radiation." Photochemistry & Photobiology 74(3): 465-70.
Young, J. M., et al. (1984). "The mouse ear inflammatory response to topical arachidonic acid." Journal of Investigative Dermatology. 82(4): 367-71.

The following description pages 53 to 56 contain preferred embodiments. Accordingly, the term "claim" as used therein refers to such a "preferred embodiment".
1. Use of a compound of formula (I) in the manufacture of a medicament for the treatment of an inflammatory disease, wherein the compound of formula (I) is: 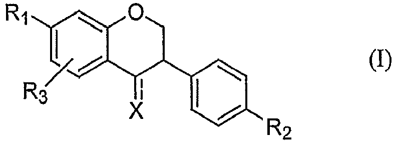 and salts thereof wherein:
   - R₁ and R₂: are independently hydroxy, alkoxy or acyloxy,
   - R₃: is hydroxy, alkoxy, alkyl or halo, and
   - X: is either =O or hydrogen and hydroxy.
2. Use according to Claim 1, wherein R₁ and R₂ are hydroxy.
3. Use according to Claim 1 or 2, wherein R₃ is methyl, bromo, chloro or hydroxy.
4. Use according to any one of claims 1 to 3, wherein X is =O and the compounds are isoflavan-4-ones of the formula (Ia): 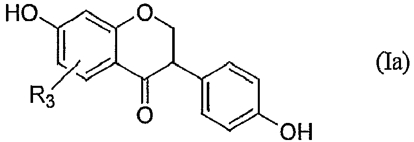 and wherein
   - R₃: is 5-allcyl, 6-halo or 8-halo,
5. Use according to claim 4, wherein R₃ is 5-methyl, 6-chloro or 8-bromo.
6. Use according to any one of claims 1 to 3, wherin X is hydrogen and hydroxy and the compounds are isoflavan-4-ols of the formula (Ib): 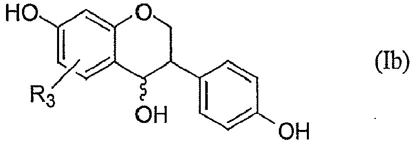 and wherein
   - R3: is 8-alkyl or 8-hydroxy.
7. Use according to claim 6, wherein R₃ is 8-methyl or 8-hydroxy.
8. Use according to claim 1, wherein the compound is an 8-substituted isoflavonoid compound of the formula (Ic): 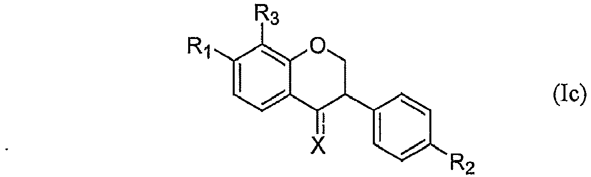 and salts thereof wherein
   - R₁ and R₂: are independently hydroxy, alkoxy or acyloxy,
   - R₃: is hydroxy, alkyl or halo, and
   - X: is either =O or hydrogen and hydroxy.
9. Use according to claim 8, wherein
   - R₁ and R₂: are hydroxy, and
   - R₃: is hydroxy, methyl or bromo.
10. Use according to claim 1 wherein the compound of formula (I) is selected from compounds 1 to 5: 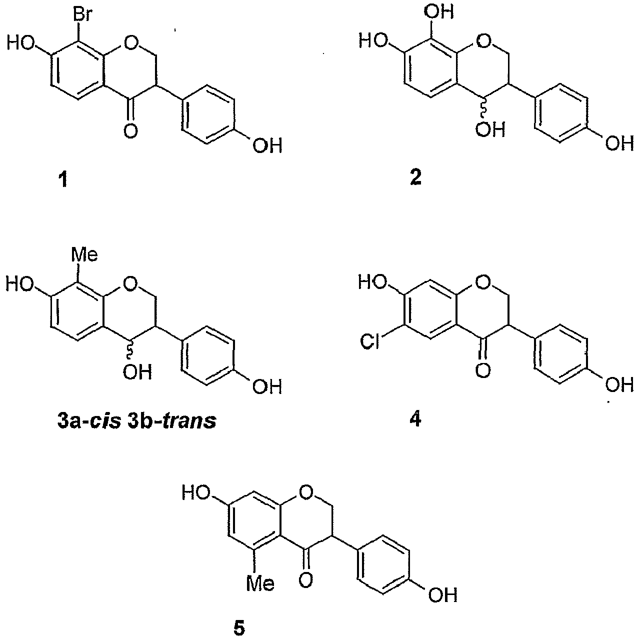 and pharmaceutically acceptable salts thereof.
11. Use according to any one of claims 1 to 10, wherein the inflammatory disease or disorder is selected from osteoarthritis, inflammatory bowel disease (ulcerative colitis and Crohn's disease), ulcerative proctitis, distal colitis, autoimmune disorders (SLE, rheumatoid arthritis, glomerulonephritis), asthma and diseases involving pulmonary inflammation, cardiovascular disorders including atherosclerosis, hypertension and lipid dyscrasias and disorders associated with estrogen receptor activation.
12. Use according to claim 11, wherein the treatment is for pain, oedema and/or erythema associated with inflammation.
13. Use according to any one of claims 1 to 12, wherein the treatment of the inflammatory disorder is absent cardiovascular side effects, is cardioprotective and/or is gut protective.
14. Use of a thromboxane synthase inhibitor for the manufacture of a medicament for the treatment of inflammation, including pain associated with inflammation.
15. Use according to claim 14, wherein the treatment is cardioprotective and/or gut protective.
16. A pharmaceutical agent comprising a compound of formula (I) according to any one of claims 1 to 10 for the treatment of inflammatory diseases and disorders or as a thromboxane synthase inhibitor.

## Claims

1. A compound of Formula (lc) having the structure: 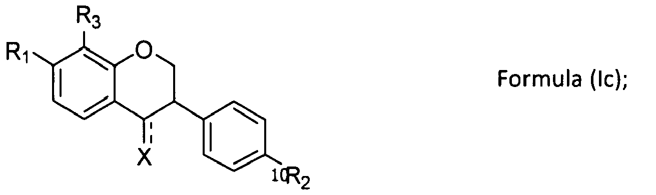 wherein:
R₁ and R₂ are independently hydroxy, alkoxy,
or acyloxy;
R₃ is hydroxy, alkyl, or halo;
X is =O;
or pharmaceutically acceptable salts thereof.

2. The compound of claim 1, wherein R₁ is hydroxy.

3. The compound of either of claims 1 or 2, wherein R₂ is hydroxy.

4. The compound of any one of claims 1-3, wherein R₃ is hydroxy, methyl or bromo.

5. The compound of claim 1, having the structure:

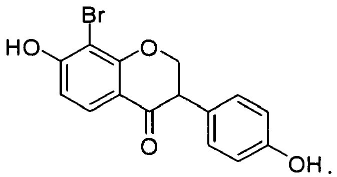

6. A pharmaceutical composition comprising a therapeutically effective amount of a compound of any one of claims 1-5, and a pharmaceutically acceptable excipient.

7. The pharmaceutical composition of claim 6 that is formulated for oral administration.

8. Use of a compound of any one of claims 1-5 for the manufacture of a medicament for the treatment of inflammation.
